# EUROPEAN PATENT SPECIFICATION

(11) **EP 2 644 209 B1**
(45) Date of publication and mention of the grant of the patent: **16.11.2022**
(21) Application number: 13161480.2
(22) Date of filing: 27.03.2013
(51) Int. Cl.: A61L 27/56, A61B 17/072, B29C 44/00

(54) **SURGICAL STAPLER INCLUDING NON-UNIFORM TISSUE THICKNESS COMPENSATOR**
CHIRURGISCHES KLAMMERGERÄT MIT GEWEBEDICKENAUSGLEICHSANORDNUNG UNEINHEITLICHER STÄRKE
AGRAFEUSE CHIRURGICALE AVEC DES AGENCEMENTS VARIABLES DE COMPENSATION D'ÉPAISSEUR DE TISSU

(30) Priority: 28.03.2012 US 201213433163
(43) Date of publication of application: 02.10.2013
(73) Proprietor: Ethicon Endo-Surgery, Inc., Cincinnati, OH 45242 (US)
(72) Inventor: Widenhouse, Tamara, Clarksville, Ohio 45113 (US); Shelton, IV Frederick E., Hillsboro, Ohio 45133 (US); Timmer, Mark D., Jersey City, New Jersey 07302 (US); Hall, Steven G., Cincinnati, Ohio 45242 (US); Timm, Richard W., Cincinnati, Ohio 45209 (US); Lang, Matthew M., Mason, Ohio 45040 (US)
(74) Representative: Carpmaels & Ransford LLP

(56) References cited:
- EP-A2- 1 815 804
- EP-A2- 2 008 595
- US-A1- 2006 085 030
- US-A1- 2008 210 738
- US-B1- 6 596 304

## Description

### BACKGROUND

The present invention relates to surgical instruments and, in various embodiments, to surgical cutting and stapling instruments and staple cartridges therefor that are designed to cut and staple tissue.

EP 1815804 describes an apparatus for supporting tissue during compression prior to the insertion of a surgical staple into the tissue. It includes a substrate having a thickness and a stress-strain profile that is designed to be complementary to the stress strain profile of the tissue, and permits the substrate to support the tissue when the surgical staple is delivered into the tissue. The substrate provides hemostasis control of the tissue when the surgical staple is delivered into the tissue.

US 2008/0210738 describes a staple cartridge for use with a stapling device that has an actuator that is selectively actuatable in an axial direction and an anvil portion that is selectively movable between open and closed positions. A cartridge body movably supports first and second staple drivers. The staple drivers each support a staple and serve to drive the staples into forming contact with the anvil upon actuation by the actuator. The final formed heights of the staples can be varied so as to apply different clamping forces and pressures to soft tissue captured within the staples. The staples can also include crowns which can be used to adjust or control the clamping force and/or pressure applied by the staples.

US 6,596,304 describes bicomposite material based on collagen for use in preventing post-operative adhesions. The material has two closely bound layers and is biocompatible, nontoxic, hemostatic and biodegradable in less than a month, and can be used in surgery to achieve hemostasis as well as to prevent post-surgical adhesion. To prepare the material, a solution of collagen or gelatin, which may contain glycerine and a hydrophilic additive such as polyethylene glycol or a polysaccharide, is poured onto an inert support to form a layer 30 µm to less than 100 µm thick. Then a polymeric porous fibrous layer is applied during gelling of the collagen or gelatin, and the resultant material is dried. The polymeric porous fibrous layer may be made of collagen or a polysaccharide, and have a density of not more than 75 mg/cm2, a pore size from 30 µm to 300 µm and a thickness of 0.2 cm to 1.5 cm.

EP 2008595 describes a surgical stapling apparatus comprising a staple cartridge containing staples, an anvil having a staple forming surface and a buttress positioned adjacent the anvil or the cartridge. The buttress includes a non-porous layer, a porous layer and a reinforcement member such as a suture or mesh.

US 2006/0085030 describes a seal element for positioning between tubular sections of tissue to be joined. The seal element includes a first material that promotes tissue ingrowth and a second material comprising a sealant at least partially surrounding the first material. The first material is a porous material, such as a foam, a mesh, a non-woven structure, or a perforated sheet. The first material is glycolide, lactide, caprolactone, trimethylene carbonate, dioxanone, or polyalkylene oxide.

### SUMMARY

The present invention provides a surgical stapling device, comprising:
a surgical staple cartridge including a deck surface and housing a plurality of surgical staples;
an anvil including a staple-forming surface and being movably supported relative to the surgical staple cartridge such that the staple-forming surface may be positioned in confronting relationship relative to the staple cartridge deck surface;
wherein the stapling device is configured to form the plurality of staples housed in the staple cartridge against the staple-forming surface of the anvil in confronting relationship relative to the staple deck surface such that the staples are formed with different formed heights; and
a tissue thickness compensator positioned between the staple forming surface of the anvil and the staple cartridge deck surface and including a non-uniform thickness measured from the staple-forming surface of the anvil to the staple cartridge deck surface.

### BRIEF DESCRIPTION OF THE DRAWINGS

The features and advantages of this invention, and the manner of attaining them, will become more apparent and the invention itself will be better understood by reference to the following description taken in conjunction with the accompanying drawings, wherein:
FIG. 1 is a perspective view of a staple cartridge comprising a rigid support portion and a compressible tissue thickness compensator for use with a surgical stapling instrument in accordance with at least one embodiment of the invention;
FIG. 2 is a partially exploded view of the staple cartridge of FIG. 1;
FIG. 3 is a fully exploded view of the staple cartridge of FIG. 1;
FIG. 4 is another exploded view of the staple cartridge of FIG. 1 without a warp covering the tissue thickness compensator;
FIG. 5 is a perspective view of a cartridge body, or support portion, of the staple cartridge of FIG. 1;
FIG. 6 is a top perspective view of a sled movable within the staple cartridge of FIG. 1 to deploy staples from the staple cartridge;
FIG. 7 is a bottom perspective view of the sled of FIG. 6;
FIG. 8 is an elevational view of the sled of FIG. 6;
FIG. 9 is a top perspective view of a driver configured to support one or more staples and to be lifted upwardly by the sled of FIG. 6 to eject the staples from the staple cartridge;
FIG. 10 is a bottom perspective view of the driver of FIG. 9;
FIG. 11 is a wrap configured to at least partially surround a compressible tissue thickness compensator of a staple cartridge;
FIG. 12 is a partial cut away view of a staple cartridge comprising a rigid support portion and a compressible tissue thickness compensator illustrated with staples being moved from an unfired position to a fired position during a first sequence;
FIG. 13 is an elevational view of the staple cartridge of FIG. 12;
FIG. 14 is a detail elevational view of the staple cartridge of FIG. 12;
FIG. 15 is a longitudinal cross-sectional view of an anvil in a closed position and a staple cartridge comprising a rigid support portion and a compressible tissue thickness compensator illustrated with staples being moved from an unfired position to a fired position during a first sequence;
FIG. 16 is another cross-sectional view of the anvil and the staple cartridge of FIG. 15 illustrating the anvil in an open position after the firing sequence has been completed;
FIG. 17 is a diagram illustrating a tissue thickness compensator which is compensating for different tissue thickness captured within different staples;
FIG. 18 is a diagram illustrating a tissue thickness compensator applying a compressive pressure to one or more vessels that have been transected by a staple line;
FIG. 19 is a diagram illustrating a circumstance wherein one or more staples have been improperly formed;
FIG. 20 is a diagram illustrating a tissue thickness compensator which could compensate for improperly formed staples;
FIG. 21 is a diagram illustrating tissue captured within a staple;
FIG. 22 is a diagram illustrating tissue and a tissue thickness compensator captured within a staple;
FIG. 23 is a diagram illustrating tissue captured within a staple;
FIG. 24 is a diagram illustrating thick tissue and a tissue thickness compensator captured within a staple;
FIG. 25 is a diagram illustrating thin tissue and a tissue thickness compensator captured within a staple;
FIG. 26 is a diagram illustrating tissue having an intermediate thickness and a tissue thickness compensator captured within a staple;
FIG. 27 is a diagram illustrating tissue having another intermediate thickness and a tissue thickness compensator captured within a staple;
FIG. 28 is a diagram illustrating thick tissue and a tissue thickness compensator captured within a staple;
FIG. 29 is a partial cross-sectional view of an end effector of a surgical stapling instrument illustrating a firing bar and staple-firing sled in a retracted, unfired position;
FIG. 30 is another partial cross-sectional view of the end effector of FIG. 29 illustrating the firing bar and the staple-firing sled in a partially advanced position;
FIG. 31 is a cross-sectional view of the end effector of FIG. 29 illustrating the firing bar in a fully advanced, or fired, position;
FIG. 32 is a cross-sectional view of the end effector of FIG. 29 illustrating the firing bar in a retracted position after being fired and the staple-firing sled left in its fully fired position;
FIG. 33 is a detail view of the firing bar in the retracted position of FIG. 32;
FIG. 34 is a partial cross-sectional view of an end effector of a surgical stapling instrument including a staple cartridge comprising a tissue thickness compensator and staples at least partially positioned therein;
FIG. 35 is another partial cross-sectional view of the end effector of FIG. 34 illustrating the staples at least partially moved and/or rotated relative to an anvil positioned opposite the staple cartridge;
FIG. 36 is partial cut-away view of a staple cartridge comprising staple drivers having different heights in accordance with at least one embodiment;
FIG. 36A is a diagram illustrating the staple drivers of FIG. 36 and staples having different unfired heights supported thereon;
FIG. 37 is a diagram illustrating a tissue thickness compensator comprising a varying thickness, staple drivers having different heights, and staples having different unformed heights;
FIG. 38 is a diagram illustrating the staples and the tissue thickness compensator of FIG. 37 implanted to tissue;
FIG. 39 is a partial cross-sectional view of a staple cartridge comprising a tissue thickness compensator comprising a varying thickness in accordance with at least one embodiment;
FIG. 40 is a cross-sectional view of an end effector of a surgical stapling instrument comprising staple drivers having different heights and a contoured deck surface in accordance with at least one embodiment;
FIG. 41 is a cross-sectional view of an end effector of a surgical stapling instrument comprising staple drivers having different heights and a stepped deck surface in accordance with at least one embodiment;
FIG. 42 is a partial cross-sectional perspective view of a mold embodiment for forming a tissue thickness compensator embodiment;
FIG. 43 is a partial cross-sectional perspective view of another mold embodiment for forming another tissue thickness compensator embodiment;
FIG. 44 is a partial cross-sectional perspective view of another mold embodiment for forming another tissue thickness compensator embodiment;
FIG. 45 is a partial cross-sectional perspective view of a film embodiment for a tissue thickness compensator embodiment;
FIG. 46 is another partial cross-sectional perspective view of the film embodiment of FIG. 45;
FIG. 47 is a partial cross-sectional perspective view of another mold embodiment for forming another tissue thickness compensator embodiment using the film embodiment of FIGS. 45 and 46;
FIG. 48 is a cross-sectional view of another mold arrangement for forming another tissue thickness compensator embodiment;
FIG. 49 is a cross-sectional view of an anvil embodiment of a surgical stapling device with a tissue thickness compensator embodiment attached to the staple forming surface thereof;
FIG. 50 is a cross-sectional view of another mold arrangement for forming two other tissue thickness compensator embodiments;
FIG. 51 is a cross-sectional view of another mold arrangement and surgical staple cartridge for forming tissue thickness compensator embodiments corresponding to portions of the staple cartridge deck;
FIG. 52 is a cross-sectional view of an anvil embodiment of a surgical stapling device with tissue thickness compensator embodiments attached to portions thereof;
FIG. 53 is a cross-sectional view of another mold arrangement and surgical staple cartridge for forming tissue thickness compensator embodiments corresponding to portions of the staple cartridge deck;
FIG. 54 is a cross-sectional view of the surgical stapling cartridge with tissue thickness compensator embodiments formed thereon and used in connection with an anvil embodiment of the surgical stapling device; and
FIG. 55 is a cross-sectional view of the surgical stapling device of FIG. 54 stapling tissue that has a varying tissue thickness profile.

### DETAILED DESCRIPTION

Any of the methods disclosed or claimed herein for manufacturing, forming or otherwise producing an article or product, may be employed to manufacture, form or otherwise produce all or part of the article or product in question, and where such a method is employed to manufacture, form or otherwise produce part of the article or product in question, the remainder of the article or product may be produced in any way, including by employing any of the other methods disclosed and claimed herein for manufacturing, forming or otherwise producing the article or product, and the various parts so produced may be combined in any manner. Similarly, any article or product disclosed or claimed herein may exist alone, or in combination with, or as an integral part of any other article or product so disclosed with which it is compatible. Thus, the particular features, structures, or characteristics illustrated or described in connection with one article, product or method may be combined, in whole or in part, with the features structures, or characteristics of one or more other compatible articles, products or methods without limitation. Such modifications and variations are included within the scope of the present invention.

Where it is disclosed herein, either with reference to a particular figure or otherwise, that a certain embodiment of the invention or a certain article, product or method may comprise certain structures, characteristics or features, it will be understood by the reader that this signifies that those structures, characteristics or features may be embodied in the article, product or method in question in any compatible combination. In particular, such a disclosure of a number of optional structures, characteristics or features shall be understood also to disclose all of those structures, characteristics or features in combination, except in the case of structures, characteristics or features that are disclosed as alternatives to one another. Where such structures, characteristics or features are disclosed as alternatives to one another, this shall be understood to disclose those alternatives as being substitutions for each other.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications, including in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongated shaft of a surgical instrument can be advanced.

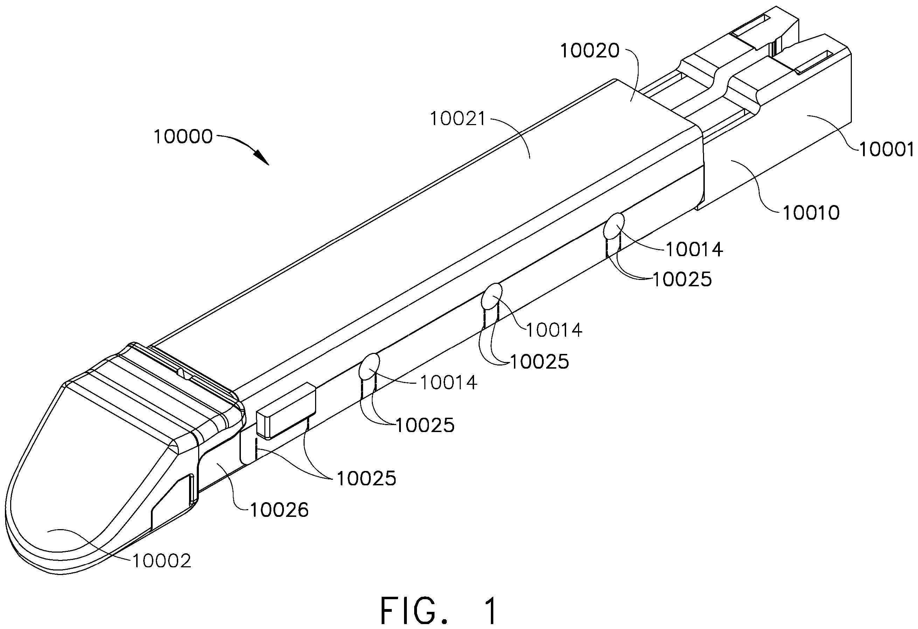

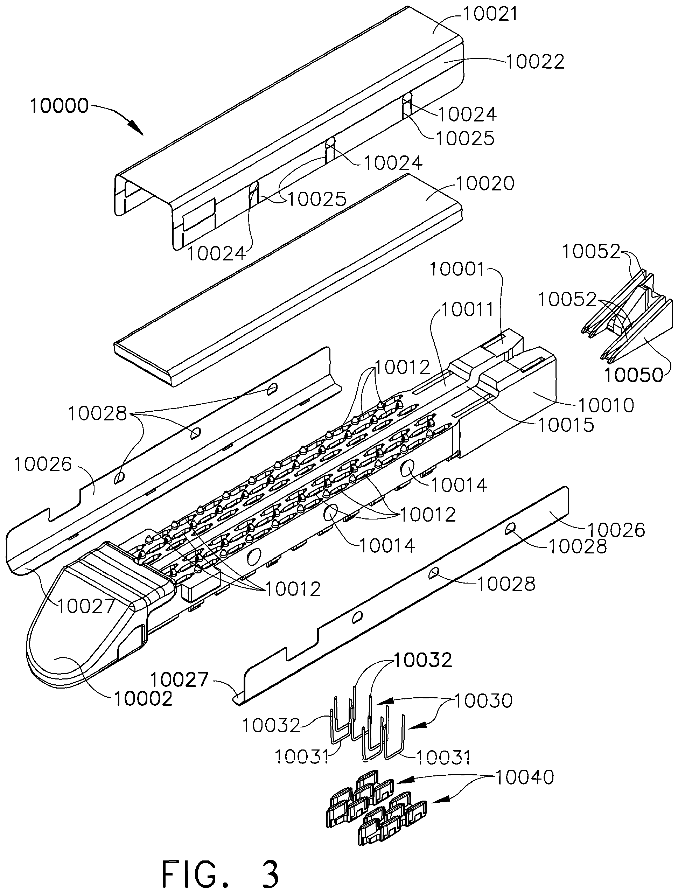

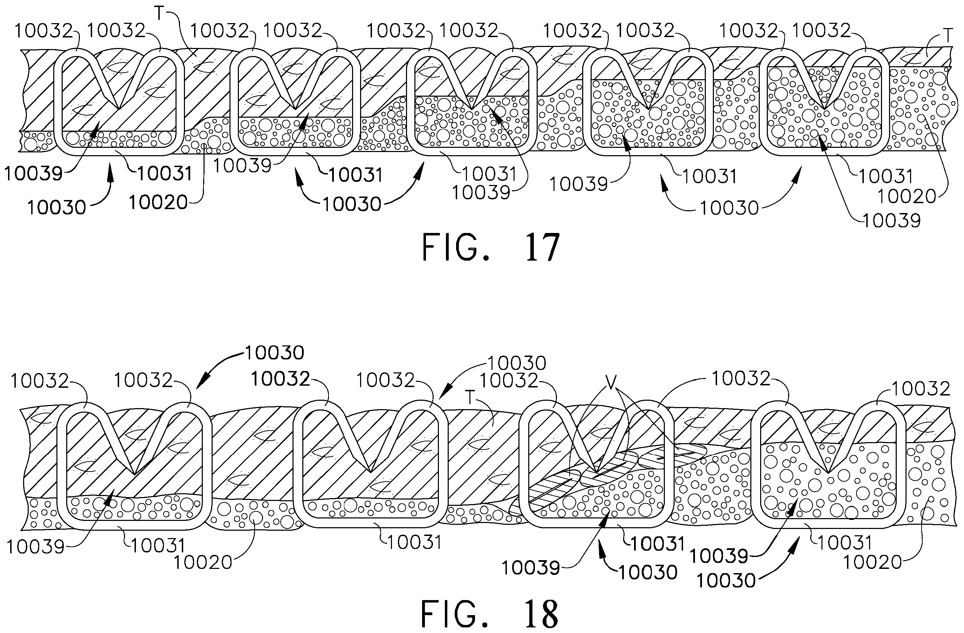

As described herein, a staple cartridge can comprise means for compensating for the thickness of the tissue captured within the staples deployed from the staple cartridge. Referring to FIG. 1, a staple cartridge, such as staple cartridge 10000, for example, can include a rigid first portion, such as support portion 10010, for example, and a compressible second portion, such as tissue thickness compensator 10020, for example. Referring primarily to FIG. 3, the support portion 10010 can comprise a cartridge body, a top deck surface 10011, and a plurality of staple cavities 10012 wherein, similar to the above, each staple cavity 10012 can define an opening in the deck surface 10011. A staple 10030, for example, can be removably positioned in each staple cavity 10012. For example, referring primarily to FIG. 17 and as described in greater detail below, each staple 10030 can comprise a base 10031 and one or more legs 10032 extending from the base 10031. Prior to the staples 10030 being deployed, as also described in greater detail below, the bases 10031 of the staples 10030 can be supported by staple drivers positioned within the support portion 10010 and, concurrently, the legs 10032 of the staples 10030 can be at least partially contained within the staple cavities 10012. The staples 10030 can be deployed between an unfired position and a fired position such that the legs 10032 move through the tissue thickness compensator 10020, penetrate through a top surface of the tissue thickness compensator 10020, penetrate the tissue T, and contact an anvil positioned opposite the staple cartridge 10000. As the legs 10032 are deformed against the anvil, the legs 10032 of each staple 10030 can capture a portion of the tissue thickness compensator 10020 and a portion of the tissue T within each staple 10030 and apply a compressive force to the tissue. Further to the above, the legs 10032 of each staple 10030 can be deformed downwardly toward the base 10031 of the staple to form a staple entrapment area 10039 in which the tissue T and the tissue thickness compensator 10020 can be captured. In various circumstances, the staple entrapment area 10039 can be defined between the inner surfaces of the deformed legs 10032 and the inner surface of the base 10031. The size of the entrapment area for a staple can depend on several factors such as the length of the legs, the diameter of the legs, the width of the base, and/or the extent in which the legs are deformed, for example.

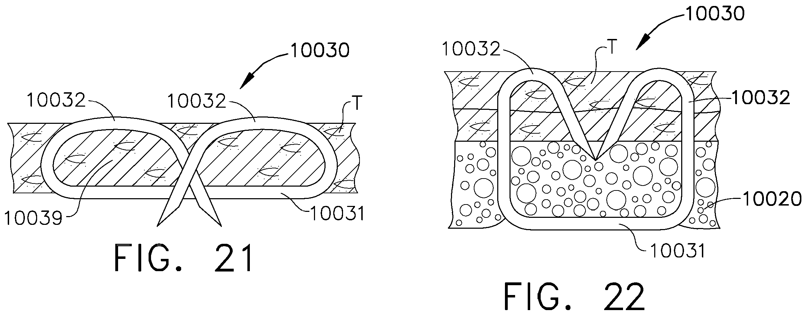

Previously, a surgeon was often required to select the appropriate staples having the appropriate staple height for the tissue being stapled. For example, a surgeon could select tall staples for use with thick tissue and short staples for use with thin tissue. In some circumstances, however, the tissue being stapled did not have a consistent thickness and, thus, some staples were unable to achieve the desired fired configuration. For example, FIG. 21 illustrates a tall staple used in thin tissue. Referring now to FIG. 22, when a tissue thickness compensator, such as tissue thickness compensator 10020, for example, is used with thin tissue, for example, the larger staple may be formed to a desired fired configuration.

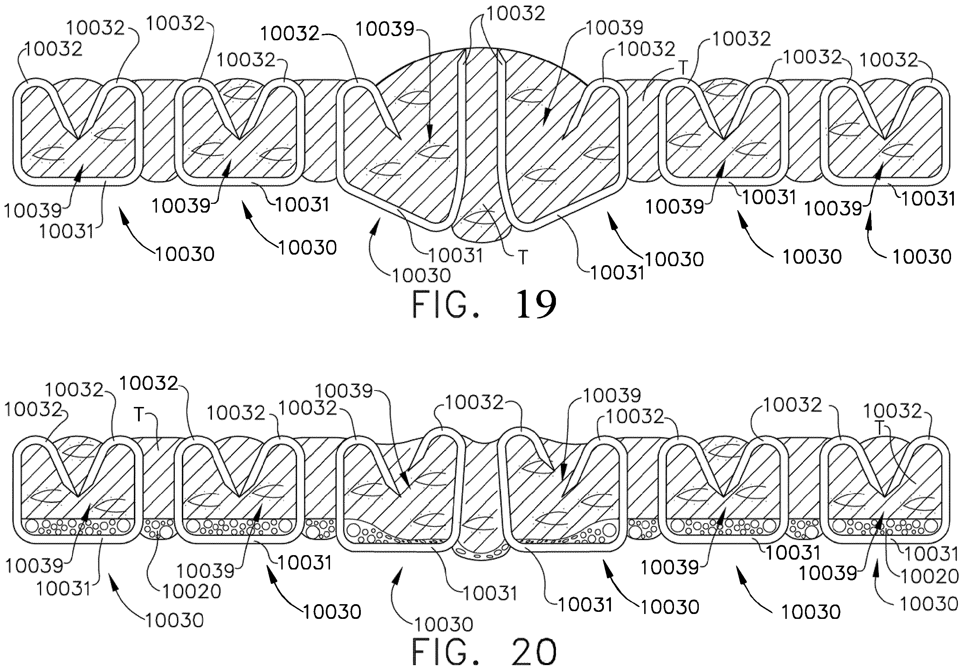

Owing to the compressibility of the tissue thickness compensator, the tissue thickness compensator can compensate for the thickness of the tissue captured within each staple. More particularly, referring now to FIGS. 17 and 18, a tissue thickness compensator, such as tissue thickness compensator 10020, for example, can consume larger and/or smaller portions of the staple entrapment area 10039 of each staple 10030 depending on the thickness and/or type of tissue contained within the staple entrapment area 10039. For example, if thinner tissue T is captured within a staple 10030, the tissue thickness compensator 10020 can consume a larger portion of the staple entrapment area 10039 as compared to circumstances where thicker tissue T is captured within the staple 10030. Correspondingly, if thicker tissue T is captured within a staple 10030, the tissue thickness compensator 10020 can consume a smaller portion of the staple entrapment area 10039 as compared to the circumstances where thinner tissue T is captured within the staple 10030. In this way, the tissue thickness compensator can compensate for thinner tissue and/or thicker tissue and assure that a compressive pressure is applied to the tissue irrespective, or at least substantially irrespective, of the tissue thickness captured within the staples. In addition to the above, the tissue thickness compensator 10020 can compensate for different types, or compressibilities, of tissues captured within different staples 10030. Referring now to FIG. 18, the tissue thickness compensator 10020 can apply a compressive force to vascular tissue T which can include vessels V and, as a result, restrict the flow of blood through the less compressible vessels V while still applying a desired compressive pressure to the surrounding tissue T. In various circumstances, further to the above, the tissue thickness compensator 10020 can also compensate for malformed staples. Referring to FIG. 19, the malformation of various staples 10030 can result in larger staple entrapment areas 10039 being defined within such staples. Owing to the resiliency of the tissue thickness compensator 10020, referring now to FIG. 20, the tissue thickness compensator 10020 positioned within malformed staples 10030 may still apply a sufficient compressive pressure to the tissue T even though the staple entrapment areas 10039 defined within such malformed staples 10030 may be enlarged. In various circumstances, the tissue thickness compensator 10020 located intermediate adjacent staples 10030 can be biased against the tissue T by properly-formed staples 10030 surrounding a malformed staple 10030 and, as a result, apply a compressive pressure to the tissue surrounding and/or captured within the malformed staple 10030, for example. In various circumstances, a tissue thickness compensator can compensate for different tissue densities which can arise due to calcifications, fibrous areas, and/or tissue that has been previously stapled or treated, for example.

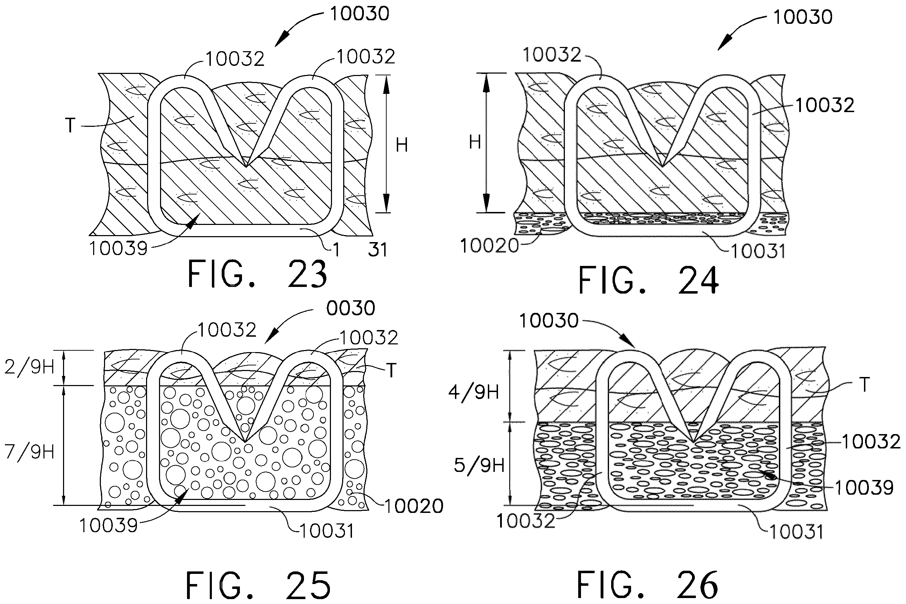

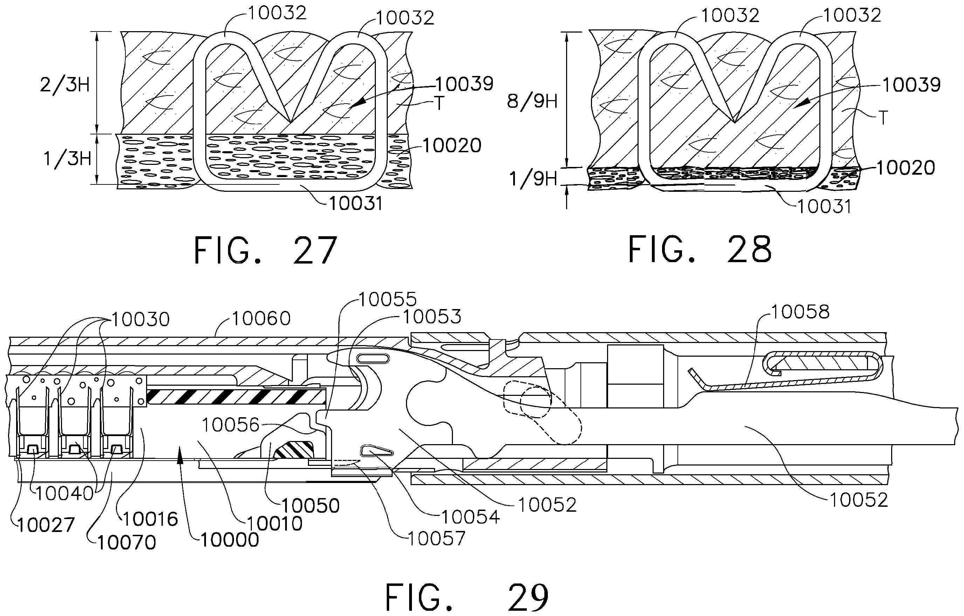

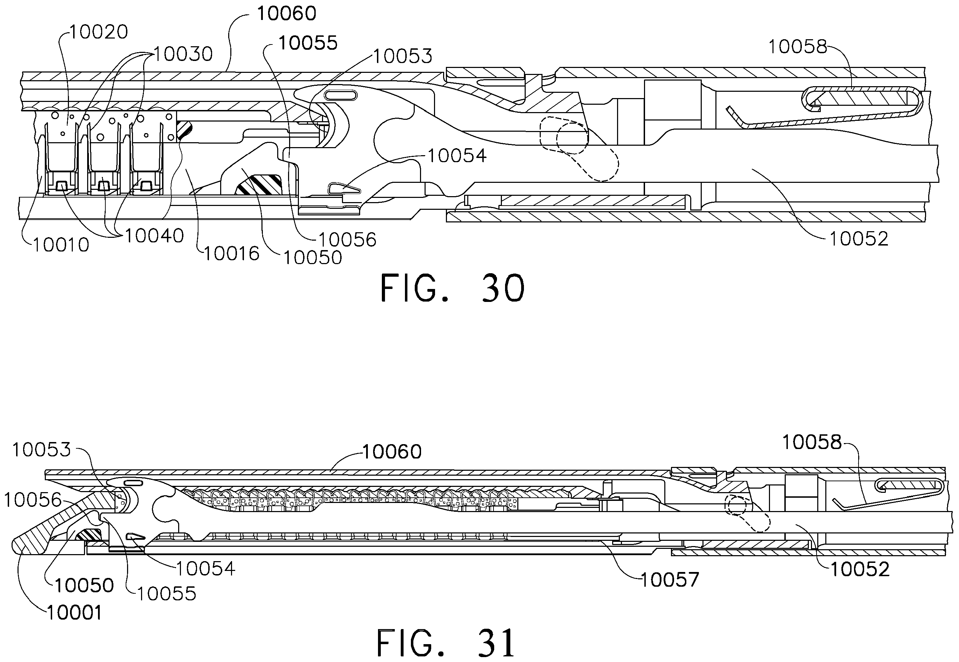

A fixed, or unchangeable, tissue gap can be defined between the support portion and the anvil and, as a result, the staples may be deformed to a predetermined height regardless of the thickness of the tissue captured within the staples. When a tissue thickness compensator is used in such cases, the tissue thickness compensator can adapt to the tissue captured between the anvil and the support portion staple cartridge and, owing to the resiliency of the tissue thickness compensator, the tissue thickness compensator can apply an additional compressive pressure to the tissue. Referring now to FIGS. 23-28, a staple 10030 has been formed to a predefined height H. With regard to FIG. 23, a tissue thickness compensator has not been utilized and the tissue T consumes the entirety of the staple entrapment area 10039. With regard to FIG. 30, a portion of a tissue thickness compensator 10020 has been captured within the staple 10030, compressed the tissue T, and consumed at least a portion of the staple entrapment area 10039. Referring now to FIG. 25, thin tissue T has been captured within the staple 10030. In this embodiment, the compressed tissue T has a height of approximately 2/9H and the compressed tissue thickness compensator 10020 has a height of approximately 7/9H, for example. Referring now to FIG. 26, tissue T having an intermediate thickness has been captured within the staple 10030. In this embodiment, the compressed tissue T has a height of approximately 4/9H and the compressed tissue thickness compensator 10020 has a height of approximately 5/9H, for example. Referring now to FIG. 27, tissue T having an intermediate thickness has been captured within the staple 10030. In this embodiment, the compressed tissue T has a height of approximately 2/3H and the compressed tissue thickness compensator 10020 has a height of approximately 1/3H, for example. Referring now to FIG. 26, thick tissue T has been captured within the staple 10030. In this embodiment, the compressed tissue T has a height of approximately 8/9H and the compressed tissue thickness compensator 10020 has a height of approximately 1/9H, for example. In various circumstances, the tissue thickness compensator can comprise a compressed height which comprises approximately 10% of the staple entrapment height, approximately 20% of the staple entrapment height, approximately 30% of the staple entrapment height, approximately 40% of the staple entrapment height, approximately 50% of the staple entrapment height, approximately 60% of the staple entrapment height, approximately 70% of the staple entrapment height, approximately 80% of the staple entrapment height, and/or approximately 90% of the staple entrapment height, for example.

The staples 10030 can comprise any suitable unformed height. The staples 10030 can comprise an unformed height between approximately 2 mm and approximately 4.8 mm, for example. The staples 10030 can comprise an unformed height of approximately 2.0 mm, approximately 2.5 mm, approximately 3.0 mm, approximately 3.4 mm, approximately 3.5 mm, approximately 3.8 mm, approximately 4.0 mm, approximately 4.1 mm, and/or approximately 4.8 mm, for example. The height H to which the staples can be deformed can be dictated by the distance between the deck surface 10011 of the support portion 10010 and the opposing anvil. The distance between the deck surface 10011 and the tissue-contacting surface of the anvil can be approximately 0.097" (2.46 mm), for example. The height H can also be dictated by the depth of the forming pockets defined within the anvil. The forming pockets can have a depth measured from the tissue-contacting surface, for example. Optionally, as described in greater detail below, the staple cartridge 10000 can further comprise staple drivers which can lift the staples 10030 toward the anvil and, lift, or "overdrive", the staples above the deck surface 10011. In such cases, the height H to which the staples 10030 are formed can also be dictated by the distance in which the staples 10030 are overdriven. For example, the staples 10030 can be overdriven by approximately 0.028" (0.71 mm), for example, and can result in the staples 10030 being formed to a height of approximately 0.189" (4.8 mm), for example. The staples 10030 can be formed to a height of approximately 0.8 mm, approximately 1.0 mm, approximately 1.5 mm, approximately 1.8 mm, approximately 2.0 mm, and/or approximately 2.25 mm, for example. The staples can be formed to a height between approximately 2.25 mm and approximately 3.0 mm, for example. Further to the above, the height of the staple entrapment area of a staple can be determined by the formed height of the staple and the width, or diameter, of the wire comprising the staple. The height of the staple entrapment area 10039 of a staple 10030 can comprise the formed height H of the staple less two diameter widths of the wire. The staple wire can comprise a diameter of approximately 0.0089" (0.226 mm), for example. The staple wire can comprise a diameter between eeapproximately 0.0069" (0.175 mm) and approximately 0.0119" (0.302 mm), for example. For example, the formed height H of a staple 10030 can be approximately 0.189" (4.8 mm) and the staple wire diameter can be approximately 0.0089" (0.226 mm) resulting in a staple entrapment height of approximately 0.171" (4.34 mm), for example.

Further to the above, the tissue thickness compensator can comprise an uncompressed, or pre-deployed, height and can be configured to deform to one of a plurality of compressed heights. The tissue thickness compensator can comprise an uncompressed height of approximately 0.125" (3.18 mm), for example. The tissue thickness compensator can comprise an uncompressed height of greater than or equal to approximately 0.080" (2.03 mm), for example. The tissue thickness compensator can comprise an uncompressed, or pre-deployed, height which is greater than the unfired height of the staples. The uncompressed, or pre-deployed, height of the tissue thickness compensator can be approximately 10% taller, approximately 20% taller, approximately 30% taller, approximately 40% taller, approximately 50% taller, approximately 60% taller, approximately 70% taller, approximately 80% taller, approximately 90% taller, and/or approximately 100% taller than the unfired height of the staples, for example. The uncompressed, or pre-deployed, height of the tissue thickness compensator can be up to approximately 100% taller than the unfired height of the staples, for example. The uncompressed, or pre-deployed, height of the tissue thickness compensator can be over 100% taller than the unfired height of the staples, for example. The tissue thickness compensator can comprise an uncompressed height which is equal to the unfired height of the staples. The tissue thickness compensator can comprise an uncompressed height which is less than the unfired height of the staples. The uncompressed, or pre-deployed, height of the thickness compensator can be approximately 10% shorter, approximately 20% shorter, approximately 30% shorter, approximately 40% shorter, approximately 50% shorter, approximately 60% shorter, approximately 70% shorter, approximately 80% shorter, and/or approximately 90% shorter than the unfired height of the staples, for example. The compressible second portion can comprise an uncompressed height which is taller than an uncompressed height of the tissue T being stapled. The tissue thickness compensator can comprise an uncompressed height which is equal to an uncompressed height of the tissue T being stapled. The tissue thickness compensator can comprise an uncompressed height which is shorter than an uncompressed height of the tissue T being stapled.

As described above, a tissue thickness compensator can be compressed within a plurality of formed staples regardless of whether thick tissue or thin tissue is captured within the staples. For example, the staples within a staple line, or row, can be deformed such that the staple entrapment area of each staple comprises a height of approximately 2.0 mm, for example, wherein the tissue T and the tissue thickness compensator can be compressed within this height. In certain circumstances, the tissue T can comprise a compressed height of approximately 1.75 mm within the staple entrapment area while the tissue thickness compensator can comprise a compressed height of approximately 0.25 mm within the staple entrapment area, thereby totaling the approximately 2.0 mm staple entrapment area height, for example. In certain circumstances, the tissue T can comprise a compressed height of approximately 1.50 mm within the staple entrapment area while the tissue thickness compensator can comprise a compressed height of approximately 0.50 mm within the staple entrapment area, thereby totaling the approximately 2.0 mm staple entrapment area height, for example. In certain circumstances, the tissue T can comprise a compressed height of approximately 1.25 mm within the staple entrapment area while the tissue thickness compensator can comprise a compressed height of approximately 0.75 mm within the staple entrapment area, thereby totaling the approximately 2.0 mm staple entrapment area height, for example. In certain circumstances, the tissue T can comprise a compressed height of approximately 1.0 mm within the staple entrapment area while the tissue thickness compensator can comprise a compressed height of approximately 1.0 mm within the staple entrapment area, thereby totaling the approximately 2.0 mm staple entrapment area height, for example. In certain circumstances, the tissue T can comprise a compressed height of approximately 0.75 mm within the staple entrapment area while the tissue thickness compensator can comprise a compressed height of approximately 1.25 mm within the staple entrapment area, thereby totaling the approximately 2.0 mm staple entrapment area height, for example. In certain circumstances, the tissue T can comprise a compressed height of approximately 1.50 mm within the staple entrapment area while the tissue thickness compensator can comprise a compressed height of approximately 0.50 mm within the staple entrapment area, thereby totaling the approximately 2.0 mm staple entrapment area height, for example. In certain circumstances, the tissue T can comprise a compressed height of approximately 0.25 mm within the staple entrapment area while the tissue thickness compensator can comprise a compressed height of approximately 1.75 mm within the staple entrapment area, thereby totaling the approximately 2.0 mm staple entrapment area height, for example.

Further to the above, the tissue thickness compensator can comprise an uncompressed height which is less than the fired height of the staples. The tissue thickness compensator can comprise an uncompressed height which is equal to the fired height of the staples. The tissue thickness compensator can comprise an uncompressed height which is taller than the fired height of the staples. For example, the uncompressed height of a tissue thickness compensator can comprise a thickness which is approximately 110% of the formed staple height, approximately 120% of the formed staple height, approximately 130% of the formed staple height, approximately 140% of the formed staple height, approximately 150% of the formed staple height, approximately 160% of the formed staple height, approximately 170% of the formed staple height, approximately 180% of the formed staple height, approximately 190% of the formed staple height, and/or approximately 200% of the formed staple height, for example. The tissue thickness compensator can comprise an uncompressed height which is more than twice the fired height of the staples. The tissue thickness compensator can comprise a compressed height which is from approximately 85% to approximately 150% of the formed staple height, for example. Optionally, as described above, the tissue thickness compensator can be compressed between an uncompressed thickness and a compressed thickness. The compressed thickness of a tissue thickness compensator can be approximately 10% of its uncompressed thickness, approximately 20% of its uncompressed thickness, approximately 30% of its uncompressed thickness, approximately 40% of its uncompressed thickness, approximately 50% of its uncompressed thickness, approximately 60% of its uncompressed thickness, approximately 70% of its uncompressed thickness, approximately 80% of its uncompressed thickness, and/ or approximately 90% of its uncompressed thickness, for example. The uncompressed thickness of the tissue thickness compensator can be approximately two times, approximately ten times, approximately fifty times, and/or approximately one hundred times thicker than its compressed thickness, for example. The compressed thickness of the tissue thickness compensator can be between approximately 60% and approximately 99% of its uncompressed thickness. The uncompressed thickness of the tissue thickness compensator can be at least 50% thicker than its compressed thickness. The uncompressed thickness of the tissue thickness compensator can be up to one hundred times thicker than its compressed thickness. The compressible second portion can be elastic, or at least partially elastic, and can bias the tissue T against the deformed legs of the staples. For example, the compressible second portion can resiliently expand between the tissue T and the base of the staple in order to push the tissue T against the legs of the staple. As discussed in further detail below, the tissue thickness compensator can be positioned intermediate the tissue T and the deformed staple legs. In various circumstances, as a result of the above, the tissue thickness compensator can be configured to consume any gaps within the staple entrapment area.

The tissue thickness compensator may comprise a polymeric composition. The polymeric composition may comprise one or more synthetic polymer and/or one or more non-synthetic polymer. The synthetic polymer may comprise a synthetic absorbable polymer and/or a synthetic non-absorbable polymer. The polymeric composition may comprise a biocompatible foam, for example. The biocompatible foam may comprise a porous, open cell foam and/or a porous, closed cell foam, for example. The biocompatible foam can have a uniform pore morphology or may have a gradient pore morphology (i.e. small pores gradually increasing in size to large pores across the thickness of the foam in one direction). The polymeric composition may comprise one or more of a porous scaffold, a porous matrix, a gel matrix, a hydrogel matrix, a solution matrix, a filamentous matrix, a tubular matrix, a composite matrix, a membranous matrix, a biostable polymer, and a biodegradable polymer, and combinations thereof. For example, the tissue thickness compensator may comprise a foam reinforced by a filamentous matrix or may comprise a foam having an additional hydrogel layer that expands in the presence of bodily fluids to further provide the compression on the tissue. According to the invention, a tissue thickness compensator could also be comprised of a coating on a material and/or a second or third layer that expands in the presence of bodily fluids to further provide the compression on the tissue. Such a layer could be a hydrogel that could be a synthetic and/or naturally derived material and could be either biodurable and/or biodegradable, for example. A tissue thickness compensator could be reinforced with fibrous non-woven materials or fibrous mesh type elements, for example, that can provide additional flexibility, stiffness, and/or strength. According to the invention, a tissue thickness compensator that has a porous morphology which exhibits a gradient structure such as, for example, small pores on one surface and larger pores on the other surface. Such morphology could be more optimal for tissue in-growth or hemostatic behavior. Further, the gradient could be also compositional with a varying bio-absorption profile. A short term absorption profile may be preferred to address hemostasis while a long term absorption profile may address better tissue healing without leakages.

Examples of non-synthetic polymers include, but are not limited to, lypholized polysaccharide, glycoprotein, elastin, proteoglycan, gelatin, collagen, and oxidized regenerated cellulose (ORC). Examples of synthetic absorbable polymers include, but are not limited to, poly(lactic acid) (PLA), poly(L-lactic acid) (PLLA), polycaprolactone (PCL), polyglycolic acid (PGA), poly(trimethylene carbonate) (TMC), polyethylene terephthalate (PET), polyhydroxyalkanoate (PHA), a copolymer of glycolide and ε-caprolactone (PGCL), a copolymer of glycolide and-trimethylene carbonate, poly(glycerol sebacate) (PGS), polydioxanone, poly(orthoesters), polyanhydrides, polysaccharides, poly(ester-amides), tyrosine-based polyarylates, tyrosine-based polyiminocarbonates, tyrosine-based polycarbonates, poly(D,L-lactide-urethane), poly(B-hydroxybutyrate), poly(E-caprolactone), polyethyleneglycol (PEG), poly[bis(carboxylatophenoxy) phosphazene], poly(amino acids), pseudo-poly(amino acids), absorbable polyurethanes, and combinations thereof. The polymeric composition may comprise from approximately 50% to approximately 90% by weight of the polymeric composition of PLLA and approximately 50% to approximately 10% by weight of the polymeric composition of PCL, for example. The polymeric composition may comprise approximately 70% by weight of PLLA and approximately 30% by weight of PCL, for example. The polymeric composition may comprise from approximately 55% to approximately 85% by weight of the polymeric composition of PGA and 15% to 45% by weight of the polymeric composition of PCL, for example. The polymeric composition may comprise approximately 65% by weight of PGA and approximately 35% by weight of PCL, for example. The polymeric composition may comprise from approximately 90% to approximately 95% by weight of the polymeric composition of PGA and approximately 5% to approximately 10% by weight of the polymeric composition of PLA, for example.

The synthetic absorbable polymer may comprise a bioabsorbable, biocompatible elastomeric copolymer. Suitable bioabsorbable, biocompatible elastomeric copolymers include but are not limited to copolymers of epsilon-caprolactone and glycolide (preferably having a mole ratio of epsilon-caprolactone to glycolide of from about 30:70 to about 70:30, preferably 35:65 to about 65:35, and more preferably 45:55 to 35:65); elastomeric copolymers of epsilon-caprolactone and lactide, including L-lactide, D-lactide blends thereof or lactic acid copolymers (preferably having a mole ratio of epsilon-caprolactone to lactide of from about 35:65 to about 65:35 and more preferably 45:55 to 30:70) elastomeric copolymers of p-dioxanone (1,4-dioxan-2-one) and lactide including L-lactide, D-lactide and lactic acid (preferably having a mole ratio of p-dioxanone to lactide of from about 40:60 to about 60:40); elastomeric copolymers of epsilon-caprolactone and p-dioxanone (preferably having a mole ratio of epsilon-caprolactone to p-dioxanone of from about 30:70 to about 70:30); elastomeric copolymers of p-dioxanone and trimethylene carbonate (preferably having a mole ratio of p-dioxanone to trimethylene carbonate of from about 30:70 to about 70:30); elastomeric copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. The elastomeric copolymer may be a copolymer of glycolide and epsilon-caprolactone. Alternatively, the elastomeric copolymer is a copolymer of lactide and epsilon-caprolactone.

The synthetic absorbable polymer may comprise one or more of 90/10 poly(glycolide-L-lactide) copolymer, commercially available from Ethicon, Inc. under the trade designation VICRYL (polyglactic 910), polyglycolide, commercially available from American Cyanamid Co. under the trade designation DEXON, polydioxanone, commercially available from Ethicon, Inc. under the trade designation PDS, poly(glycolide-trimethylene carbonate) random block copolymer, commercially available from American Cyanamid Co. under the trade designation MAXON, 75/25 poly(glycolide-E-caprolactone-poliglecaprolactone 25) copolymer, commercially available from Ethicon under the trade designation MONOCRYL, for example.

Examples of synthetic non-absorbable polymers include, but are not limited to, foamed polyurethane, polypropylene (PP), polyethylene (PE), polycarbonate, polyamides, such as nylon, polyvinylchloride (PVC), polymethylmetacrylate (PMMA), polystyrene (PS), polyester, polyetheretherketone (PEEK), polytetrafluoroethylene (PTFE), polytrifluorochloroethylene (PTFCE), polyvinylfluoride (PVF), fluorinated ethylene propylene (FEP), polyacetal, polysulfone, and combinations thereof. The synthetic non-absorbable polymers may include, but are not limited to, foamed elastomers and porous elastomers, such as, for example, silicone, polyisoprene, and rubber. The synthetic polymers may comprise expanded polytetrafluoroethylene (ePTFE), commercially available from W. L. Gore & Associates, Inc. under the trade designation GORE-TEX Soft Tissue Patch and co-polyetherester urethane foam commercially available from Polyganics under the trade designation NASOPORE.

The polymeric composition of a tissue thickness compensator may be characterized by percent porosity, pore size, and/or hardness, for example. The polymeric composition may have a percent porosity from approximately 30% by volume to approximately 99% by volume, for example. The polymeric composition may have a percent porosity from approximately 60% by volume to approximately 98% by volume, for example. The polymeric composition may have a percent porosity from approximately 85% by volume to approximately 97% by volume, for example. The polymeric composition may comprise approximately 70% by weight of PLLA and approximately 30% by weight of PCL, for example, and can comprise approximately 90% porosity by volume, for example. For example, as a result, the polymeric composition would comprise approximately 10% copolymer by volume. The polymeric composition may comprise approximately 65% by weight of PGA and approximately 35% by weight of PCL, for example, and can have a percent porosity from approximately 93% by volume to approximately 95% by volume, for example. The polymeric composition may comprise a greater than 85% porosity by volume. The polymeric composition may have a pore size from approximately 5 micrometers to approximately 2000 micrometers, for example. The polymeric composition may have a pore size between approximately 10 micrometers to approximately 100 micrometers, for example. For example, the polymeric composition can comprise a copolymer of PGA and PCL, for example. The polymeric composition may have a pore size between approximately 100 micrometers to approximately 1000 micrometers, for example. For example, the polymeric composition can comprise a copolymer of PLLA and PCL, for example. According to certain aspects, the hardness of a polymeric composition may be expressed in terms of the Shore Hardness, which can defined as the resistance to permanent indentation of a material as determined with a durometer, such as a Shore Durometer. In order to assess the durometer value for a given material, a pressure is applied to the material with a durometer indenter foot in accordance with ASTM procedure D2240-00, entitled, "Standard Test Method for Rubber Property-Durometer Hardness." The durometer indenter foot may be applied to the material for a sufficient period of time, such as 15 seconds, for example, wherein a reading is then taken from the appropriate scale. Depending on the type of scale being used, a reading of 0 can be obtained when the indenter foot completely penetrates the material, and a reading of 100 can be obtained when no penetration into the material occurs. This reading is dimensionless. The durometer may be determined in accordance with any suitable scale, such as Type A and/or Type OO scales, for example, in accordance with ASTM D2240-00. The polymeric composition of a tissue thickness compensator may have a Shore A hardness value from approximately 4 A to approximately 16 A, for example, which is approximately 45 OO to approximately 65 OO on the Shore OO range. For example, the polymeric composition can comprise a PLLA/PCL copolymer or a PGA/PCL copolymer, for example. The polymeric composition of a tissue thickness compensator may have a Shore A Hardness value of less than 15 A. The polymeric composition of a tissue thickness compensator may have a Shore A Hardness value of less than 10 A. The polymeric composition of a tissue thickness compensator may have a Shore A Hardness value of less than 5 A. The polymeric material may have a Shore OO composition value from approximately 35 OO to approximately 75 OO, for example.

The polymeric composition may have at least two of the above-identified properties. The polymeric composition may have at least three of the above-identified properties. The polymeric composition may have a porosity from 85% to 97% by volume, a pore size from 5 micrometers to 2000 micrometers, and a Shore A hardness value from 4 A to 16 A and Shore OO hardness value from 45 OO to 65 OO, for example. The polymeric composition may comprise 70% by weight of the polymeric composition of PLLA and 30% by weight of the polymeric composition of PCL having a porosity of 90% by volume, a pore size from 100 micrometers to 1000 micrometers, and a Shore A hardness value from 4 A to 16 A and Shore OO hardness value from 45 OO to 65 OO, for example. The polymeric composition may comprise 65% by weight of the polymeric composition of PGA and 35% by weight of the polymeric composition of PCL having a porosity from 93% to 95% by volume, a pore size from 10 micrometers to 100 micrometers, and a Shore A hardness value from 4 A to 16 A and Shore OO hardness value from 45 OO to 65 OO, for example.

The polymeric composition may comprise a pharmaceutically active agent. The polymeric composition may release a therapeutically effective amount of the pharmaceutically active agent. The pharmaceutically active agent may be released as the polymeric composition is desorbed/absorbed. The pharmaceutically active agent may be released into fluid, such as, for example, blood, passing over or through the polymeric composition. Examples of pharmaceutically active agents may include, but are not limited to, hemostatic agents and drugs, such as, for example, fibrin, thrombin, and oxidized regenerated cellulose (ORC); antiinflammatory drugs, such as, for example, diclofenac, aspirin, naproxen, sulindac, and hydrocortisone; antibiotic and antimicrobial drug or agents, such as, for example, triclosan, ionic silver, ampicillin, gentamicin, polymyxin B, chloramphenicol; and anticancer agents, such as, for example, cisplatin, mitomycin, adriamycin.

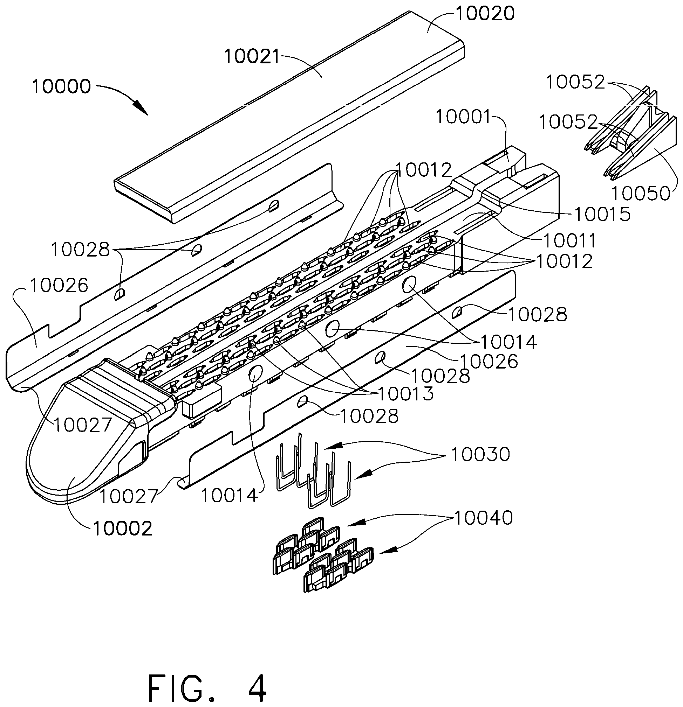

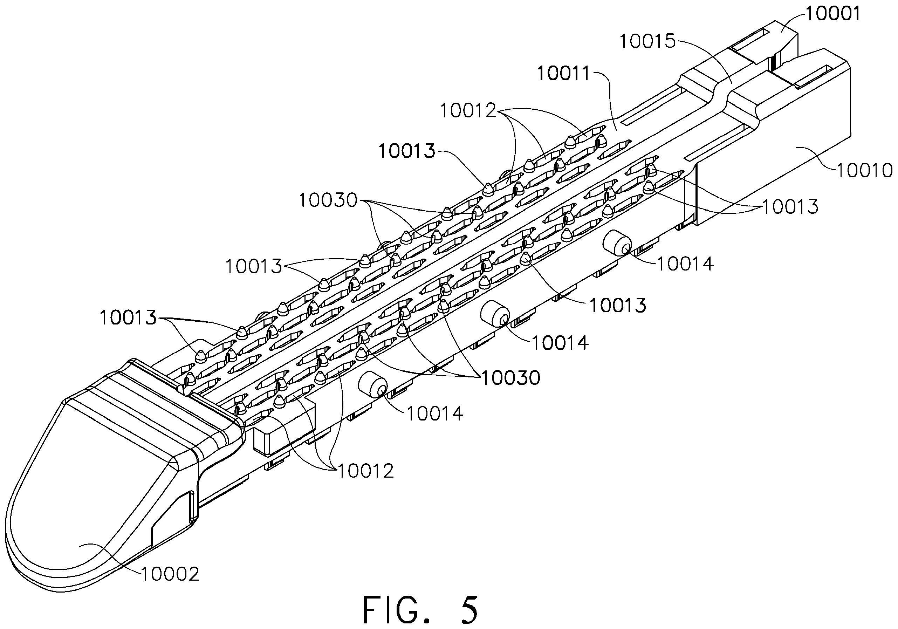

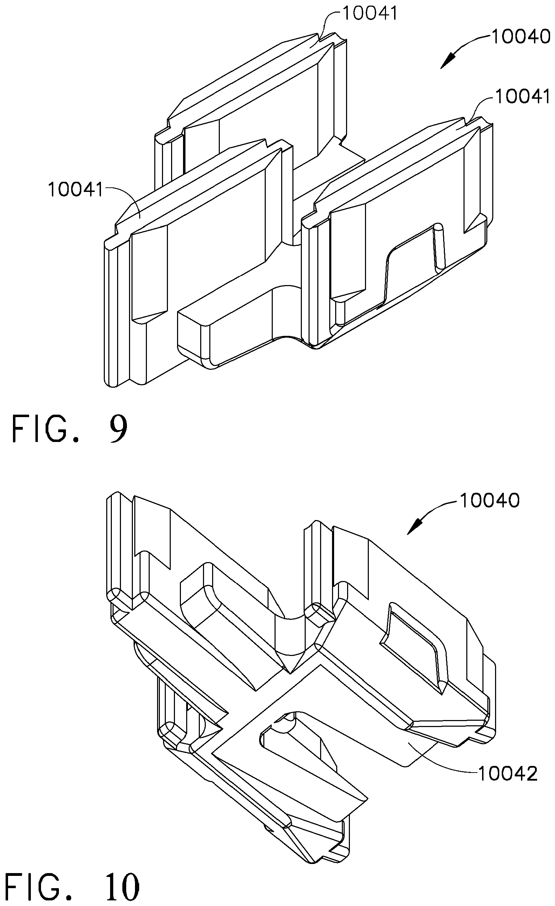

Referring now to FIG. 1, a staple cartridge, such as staple cartridge 10000, for example, can comprise a support portion 10010 and a compressible tissue thickness compensator 10020. Referring now to FIGS. 3-5, the support portion 10010 can comprise a deck surface 10011 and a plurality of staple cavities 10012 defined within the support portion 10010. Each staple cavity 10012 can be sized and configured to removably store a staple, such as a staple 10030, for example, therein. The staple cartridge 10000 can further comprise a plurality of staple drivers 10040 which can each be configured to support one or more staples 10030 within the staple cavities 10012 when the staples 10030 and the staple drivers 10040 are in their unfired positions. For example, referring primarily to FIGS. 9 and 10, each staple driver 10040 can comprise one or more cradles, or troughs, 10041, for example, which can be configured to support the staples and limit relative movement between the staples 10030 and the staple drivers 10040. Referring again to FIG. 3, the staple cartridge 10000 can further comprise a staple-firing sled 10050 which can be moved from a proximal end 10001 to a distal end 10002 of the staple cartridge in order to sequentially lift the staple drivers 10040 and the staples 10030 from their unfired positions toward an anvil positioned opposite the staple cartridge 10000. Referring primarily to FIGS. 3 and 5, each staple 10030 can comprise a base 10031 and one or more legs 10032 extending from the base 10031 wherein each staple can be at least one of substantially U-shaped and substantially V-shaped, for example. The staples 10030 can be configured such that the tips of the staple legs 10032 are recessed with respect to the deck surface 10011 of the support portion 10010 when the staples 10030 are in their unfired positions. The staples 10030 can be configured such that the tips of the staple legs 10032 are flush with respect to the deck surface 10011 of the support portion 10010 when the staples 10030 are in their unfired positions. The staples 10030 can be configured such that the tips of the staple legs 10032, or at least some portion of the staple legs 10032, extend above the deck surface 10011 of the support portion 10010 when the staples 10030 are in their unfired positions. In such cases, the staple legs 10032 can extend into and can be embedded within the tissue thickness compensator 10020 when the staples 10030 are in their unfired positions. For example, the staple legs 10032 can extend above the deck surface 10011 by approximately 0.075" (1.91 mm), for example. The staple legs 10032 can extend above the deck surface 10011 by a distance between approximately 0.025" (0.64 mm) and approximately 0.125" (3.18 mm), for example. Further to the above, the tissue thickness compensator 10020 can comprise an uncompressed thickness between approximately 0.08" (2.0 mm) and approximately 0.125" (3.18 mm), for example.

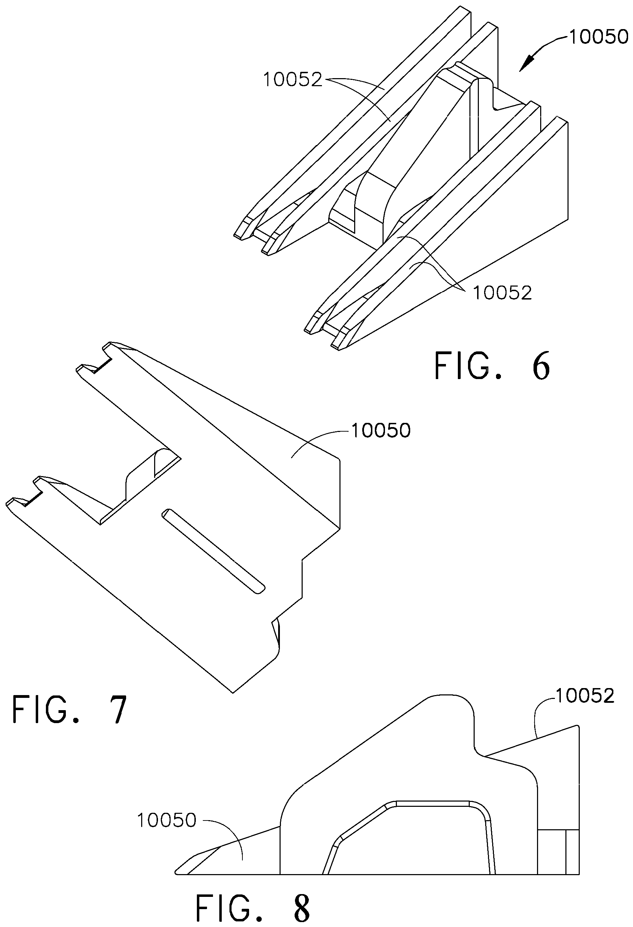

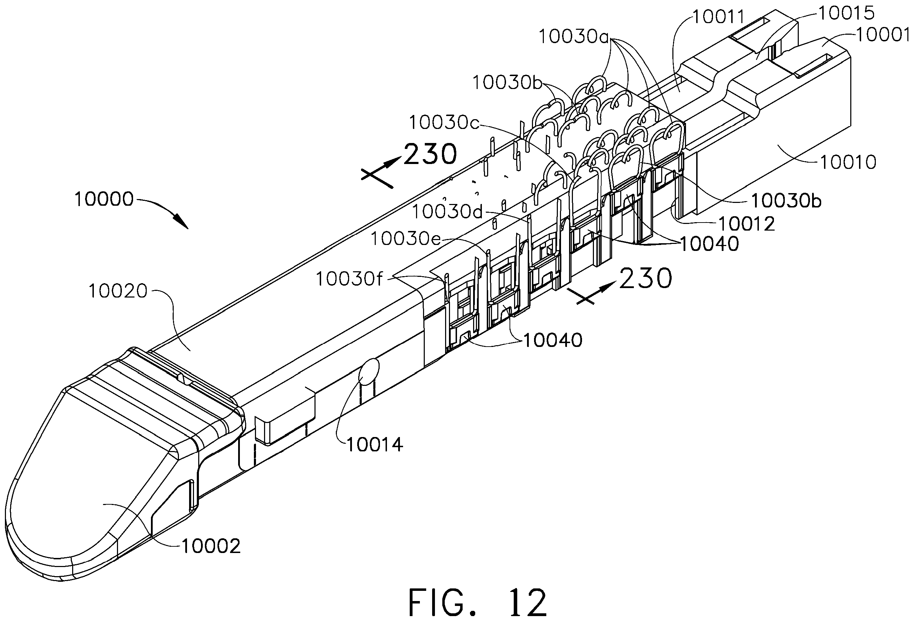

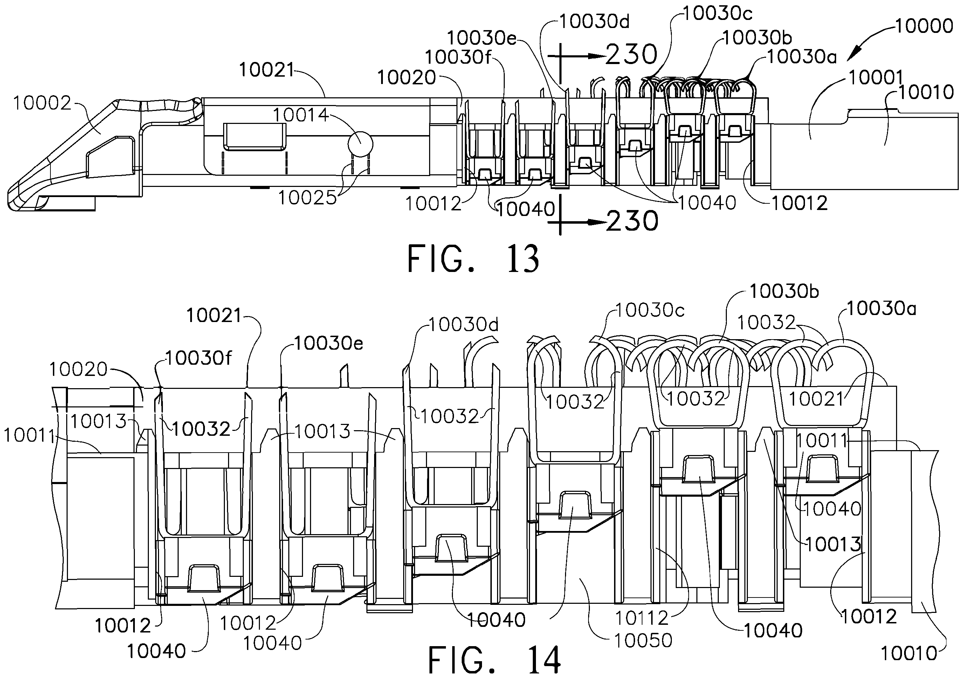

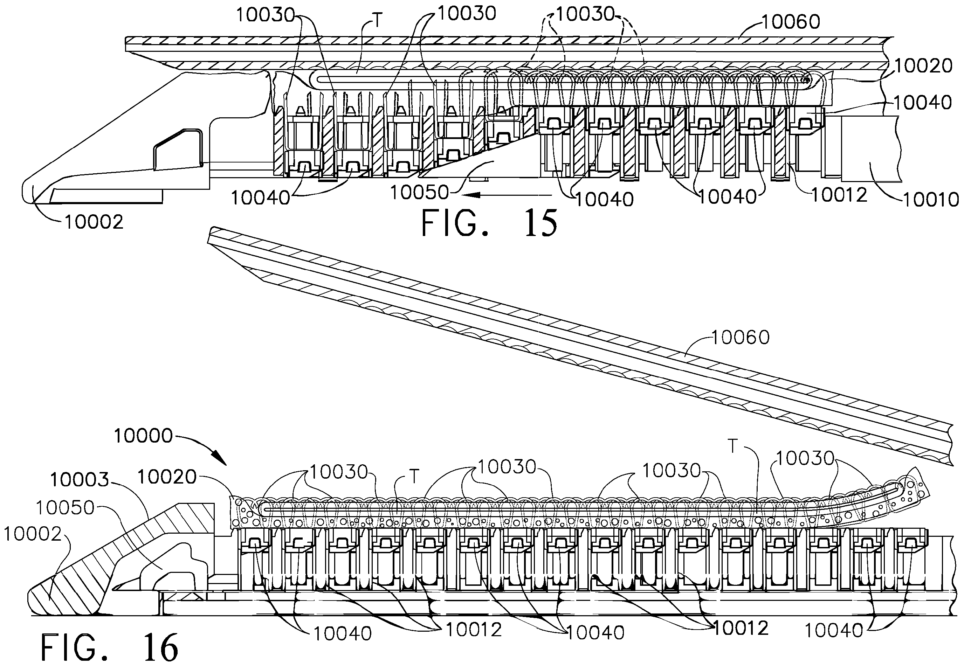

In use, further to the above and referring primarily to FIG. 15, an anvil, such as anvil, 10060, for example, can be moved into a closed position opposite the staple cartridge 10000. As described in greater detail below, the anvil 10060 can position tissue against the tissue thickness compensator 10020 and compress the tissue thickness compensator 10020 against the deck surface 10011 of the support portion 10010, for example. Once the anvil 10060 has been suitably positioned, the staples 10030 can be deployed, as also illustrated in FIG. 15. Optionally, as mentioned above, the staple-firing sled 10050 can be moved from the proximal end 10001 of the staple cartridge 10000 toward the distal end 10002, as illustrated in FIG. 16. As the sled 10050 is advanced, the sled 10050 can contact the staple drivers 10040 and lift the staple drivers 10040 upwardly within the staple cavities 10012. The sled 10050 and the staple drivers 10040 can each comprise one or more ramps, or inclined surfaces, which can co-operate to move the staple drivers 10040 upwardly from their unfired positions. For example, referring to FIGS. 6-10, each staple driver 10040 can comprise at least one inclined surface 10042 and the sled 10050 can comprise one or more inclined surfaces 10052 which can be configured such that the inclined surfaces 10052 can slide under the inclined surface 10042 as the sled 10050 is advanced distally within the staple cartridge. As the staple drivers 10040 are lifted upwardly within their respective staple cavities 10012, the staple drivers 10040 can lift the staples 10030 upwardly such that the staples 10030 can emerge from their staple cavities 10012 through openings in the staple deck 10011. During an exemplary firing sequence, referring primarily to FIGS. 12-14, the sled 10050 can first contact staple 10030a and begin to lift the staple 10030a upwardly. As the sled 10050 is advanced further distally, the sled 10050 can begin to lift staples 10030b, 10030c, 10030d, 10030e, and 10030f, and any other subsequent staples, in a sequential order. As illustrated in FIG. 14, the sled 10050 can drive the staples 10030 upwardly such that the legs 10032 of the staples contact the opposing anvil, are deformed to a desired shape, and ejected therefrom the support portion 10010. In various circumstances, the sled 10030 can move several staples upwardly at the same time as part of a firing sequence. With regard to the firing sequence illustrated in FIG. 14, the staples 10030a and 10030b have been moved into their fully fired positions and ejected from the support portion 10010, the staples 10030c and 10030d are in the process of being fired and are at least partially contained within the support portion 10010, and the staples 10030e and 10030f are still in their unfired positions.

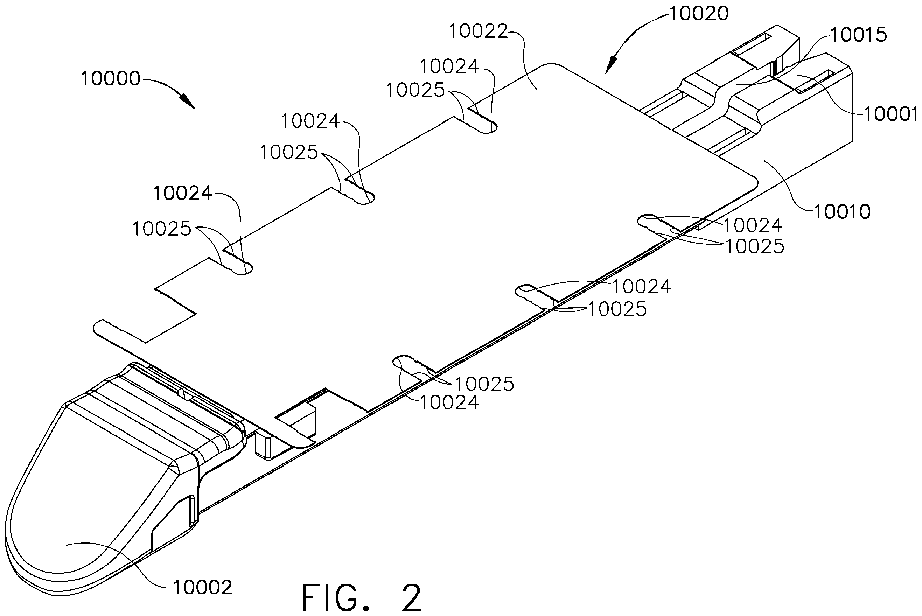

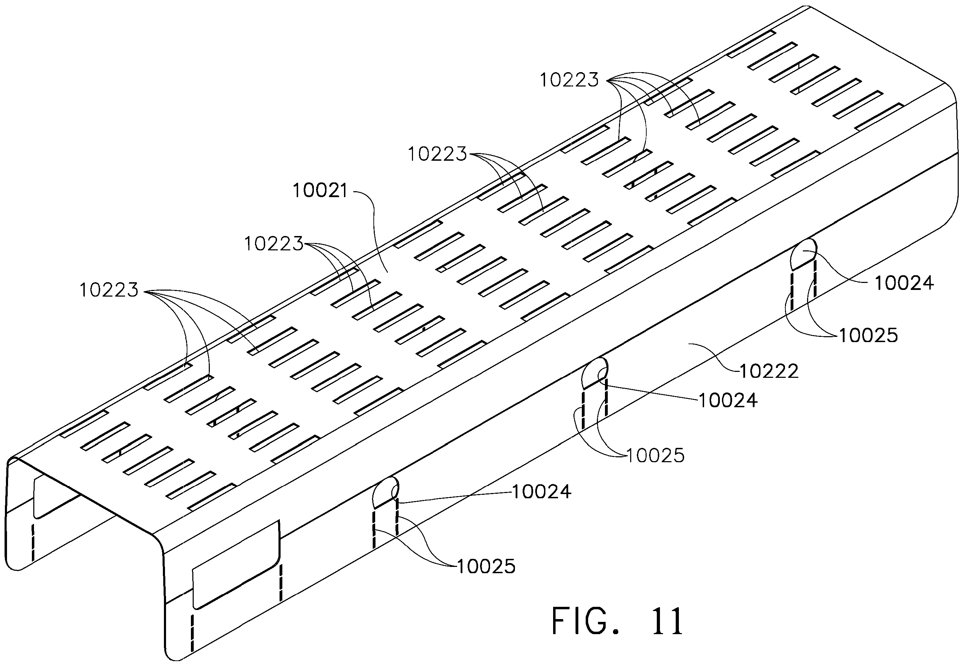

According to the invention, a tissue thickness compensator, such as tissue thickness compensator 10020, for example, can be comprised of a single sheet of material. A tissue thickness compensator can comprise a continuous sheet of material which can cover the entire top deck surface 10011 of the support portion 10010 or, alternatively, cover less than the entire deck surface 10011. The sheet of material can cover the staple cavity openings in the support portion 10010 while, alternatively, the sheet of material can comprise openings which can be aligned, or at least partially aligned, with the staple cavity openings. According to the invention, a tissue thickness compensator can be comprised of multiple layers of material. Referring now to FIG. 2, a tissue thickness compensator can comprise a compressible core and a wrap surrounding the compressible core. A wrap 10022 can be configured to releasably hold the compressible core to the support portion 10010. For example, the support portion 10010 can comprise one or more projections, such as projections 10014 (FIG. 5), for example, extending therefrom which can be received within one or more apertures and/or slots, such as apertures 10024, for example, defined in the wrap 10022. The projections 10014 and the apertures 10024 can be configured such that the projections 10014 can retain the wrap 10022 to the support portion 10010. The ends of the projections 10014 can be deformed, such as by a heat-stake process, for example, in order to enlarge the ends of the projections 10014 and, as a result, limit the relative movement between the wrap 10022 and the support portion 10010. The wrap 10022 can comprise one or more perforations 10025 which can facilitate the release of the wrap 10022 from the support portion 10010, as illustrated in FIG. 2. Referring now to FIG. 11, a tissue thickness compensator can comprise a wrap 10222 including a plurality of apertures 10223, wherein the apertures 10223 can be aligned, or at least partially aligned, with the staple cavity openings in the support portion 10010. The core of the tissue thickness compensator can also comprise apertures which are aligned, or at least partially aligned, with the apertures 10223 in the wrap 10222. Alternatively, the core of the tissue thickness compensator can comprise a continuous body and can extend underneath the apertures 10223 such that the continuous body covers the staple cavity openings in the deck surface 10011.

Optionally, as described above, a tissue thickness compensator can comprise a wrap for releasably holding a compressible core to the support portion 10010. For example, referring to FIG. 3, a staple cartridge can further comprise retainer clips 10026 which can be configured to inhibit the wrap, and the compressible core, from prematurely detaching from the support portion 10010. Optionally, each retainer clip 10026 can comprise apertures 10028 which can be configured to receive the projections 10014 extending from the support portion 10010 such that the retainer clips 10026 can be retained to the support portion 10010. The retainer clips 10026 can each comprise at least one pan portion 10027 which can extend underneath the support portion 10010 and can support and retain the staple drivers 10040 within the support portion 10010. As described above, a tissue thickness compensator can be removably attached to the support portion 10010 by the staples 10030. More particularly, as also described above, the legs of the staples 10030 can extend into the tissue thickness compensator 10020 when the staples 10030 are in their unfired position and, as a result, releasably hold the tissue thickness compensator 10020 to the support portion 10010. The legs of the staples 10030 can be in contact with the sidewalls of their respective staple cavities 10012 wherein, owing to friction between the staple legs 10032 and the sidewalls, the staples 10030 and the tissue thickness compensator 10020 can be retained in position until the staples 10030 are deployed from the staple cartridge 10000. When the staples 10030 are deployed, the tissue thickness compensator 10020 can be captured within the staples 10030 and held against the stapled tissue T. When the anvil is thereafter moved into an open position to release the tissue T, the support portion 10010 can be moved away from the tissue thickness compensator 10020 which has been fastened to the tissue. An adhesive can be utilized to removably hold the tissue thickness compensator 10020 to the support portion 10010. A two-part adhesive can be utilized wherein, a first part of the adhesive can be placed on the deck surface 10011 and a second part of the adhesive can be placed on the tissue thickness compensator 10020 such that, when the tissue thickness compensator 10020 is placed against the deck surface 10011, the first part can contact the second part to active the adhesive and detachably bond the tissue thickness compensator 10020 to the support portion 10010. Optionally, any other suitable means could be used to detachably retain the tissue thickness compensator to the support portion of a staple cartridge.

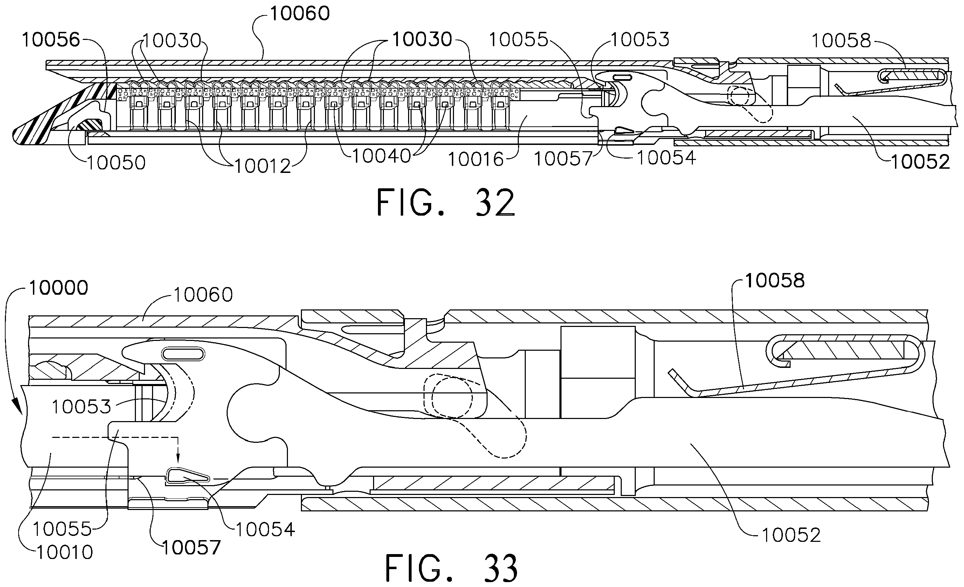

Further to the above, the sled 10050 can be advanced from the proximal end 10001 to the distal end 10002 to fully deploy all of the staples 10030 contained within the staple cartridge 10000. Referring now to FIGS. 29-33, the sled 10050 can be advanced distally within a longitudinal cavity 10016 within the support portion 10010 by a firing member, or knife bar, 10052 of a surgical stapler. In use, the staple cartridge 10000 can be inserted into a staple cartridge channel in a jaw of the surgical stapler, such as staple cartridge channel 10070, for example, and the firing member 10052 can be advanced into contact with the sled 10050, as illustrated in FIG. 29. As the sled 10050 is advanced distally by the firing member 10052, the sled 10050 can contact the proximal-most staple driver, or drivers, 10040 and fire, or eject, the staples 10030 from the cartridge body 10010, as described above. As illustrated in FIG. 29, the firing member 10052 can further comprise a cutting edge 10053 which can be advanced distally through a knife slot in the support portion 10010 as the staples 10030 are being fired. According to the invention, a corresponding knife slot can extend through the anvil positioned opposite the staple cartridge 10000 such that, the cutting edge 10053 can extend between the anvil and the support portion 10010 and incise the tissue and the tissue thickness compensator positioned therebetween. In various circumstances, the sled 10050 can be advanced distally by the firing member 10052 until the sled 10050 reaches the distal end 10002 of the staple cartridge 10000, as illustrated in FIG. 31. At such point, the firing member 10052 can be retracted proximally. The sled 10050 can be retracted proximally with the firing member 10052 but referring now to FIG. 32, the sled 10050 can be left behind in the distal end 10002 of the staple cartridge 10000 when the firing member 10052 is retracted. Once the firing member 10052 has been sufficiently retracted, the anvil can be re-opened, the tissue thickness compensator 10020 can be detached from the support portion 10010, and the remaining non-implanted portion of the expended staple cartridge 10000, including the support portion 10010, can be removed from the staple cartridge channel 10070.

After the expended staple cartridge 10000 has been removed from the staple cartridge channel, further to the above, a new staple cartridge 10000, or any other suitable staple cartridge, can be inserted into the staple cartridge channel 10070. Further to the above, the staple cartridge channel 10070, the firing member 10052, and/or the staple cartridge 10000 can comprise cooperating features which can prevent the firing member 10052 from being advanced distally a second, or subsequent, time without a new, or unfired, staple cartridge 10000 positioned in the staple cartridge channel 10070. More particularly, referring again to FIG. 29, as the firing member 10052 is advanced into contact with the sled 10050 and, when the sled 10050 is in its proximal unfired position, a support nose 10055 of the firing member10052 can be positioned on and/or over a support ledge 10056 on the sled 10050 such that the firing member 10052 is held in a sufficient upward position to prevent a lock, or beam, 10054 extending from the firing member 10052 from dropping into a lock recess defined within the staple cartridge channel. As the lock 10054 will not drop into the lock recess, in such circumstances, the lock 10054 may not abut a distal sidewall 10057 of the lock recess as the firing member 10052 is advanced. As the firing member 10052 pushes the sled 10050 distally, the firing member 10052 can be supported in its upward firing position owing to the support nose 10055 resting on the support ledge 10056. When the firing member 10052 is retracted relative to the sled 10050, as discussed above and illustrated in FIG. 32, the firing member 10052 can drop downwardly from its upward position as the support nose 10055 is no longer resting on the support ledge 10056 of the sled 10050. For example, the surgical staple can comprise a spring 10058, and/or any other suitable biasing element, which can be configured to bias the firing member 10052 into its downward position. Once the firing member 10052 has been completely retracted, as illustrated in FIG. 33, the firing member 10052 cannot be advanced distally through the spent staple cartridge 10000 once again. More particularly, the firing member 10052 can't be held in its upper position by the sled 10050 as the sled 10050, at this point in the operating sequence, has been left behind at the distal end 10002 of the staple cartridge 10000. Thus, as mentioned above, in the event that the firing member 10052 is advanced once again without replacing the staple cartridge, the lock beam 10054 will contact the sidewall 10057 of the lock recess which will prevent the firing member 10052 from being advanced distally into the staple cartridge 10000 once again. Stated another way, once the spent staple cartridge 10000 has been replaced with a new staple cartridge, the new staple cartridge will have a proximally-positioned sled 10050 which can hold the firing member 10052 in its upper position and allow the firing member 10052 to be advanced distally once again.

As described above, the sled 10050 can be configured to move the staple drivers 10040 between a first, unfired position and a second, fired position in order to eject staples 10030 from the support portion 10010. The staple drivers 10040 can be contained within the staple cavities 10012 after the staples 10030 have been ejected from the support portion 10010. The support portion 10010 can comprise one or more retention features which can be configured to block the staple drivers 10040 from being ejected from, or falling out of, the staple cavities 10012. Alternatively, the sled 10050 can be configured to eject the staple drivers 10040 from the support portion 10010 with the staples 10030. For example, the staple drivers 10040 can be comprised of a bioabsorbable and/or biocompatible material, such as Ultem, for example. The staple drivers can be attached to the staples 10030. For example, a staple driver can be molded over and/or around the base of each staple 10030 such that the driver is integrally formed with the staple.

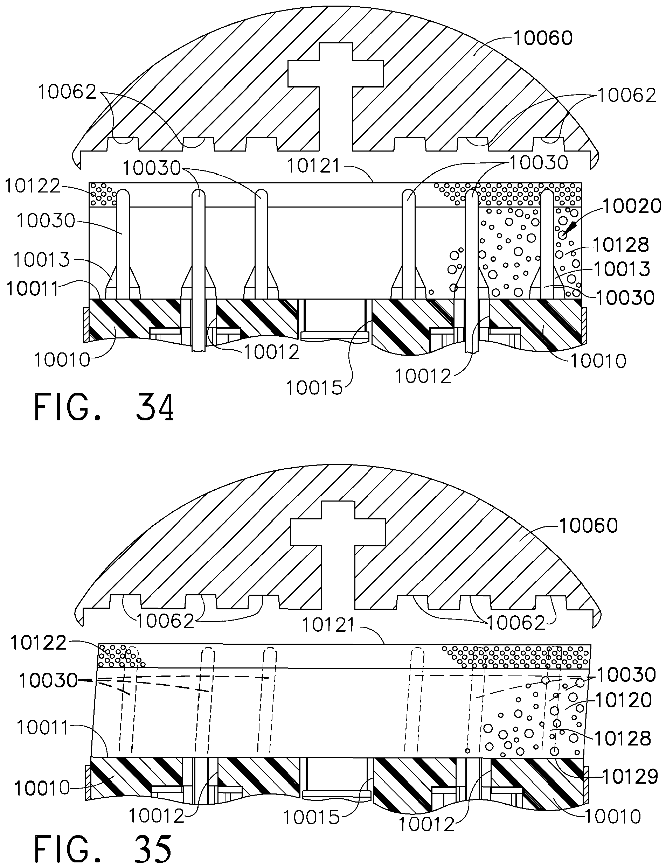

Referring again to FIGS. 34 and 35, a tissue thickness compensator, such as tissue thickness compensator 10120, for example, can comprise a core 10128 and a skin 10122. The skin 10122 and the compressible core 10128 can be comprised of different materials or, alternatively, of the same material. In either event, the skin 10122 can have a higher density than the core 10128. In circumstances where the skin 10122 comprises the top of the tissue thickness compensator 10120, the tips of the staple legs 10032 can be embedded in the skin 10122. Where a skin comprises the bottom of the tissue thickness compensator 10120, the staple legs 10032 can extend through the skin and into the core. In either event, the skin of the tissue thickness compensator can assist in holding the staple legs 10032 in alignment with the forming pockets 10062 of the anvil 10060. The skin 10122 can comprise a density which is approximately 10% greater than the density of the core 10128, approximately 20% greater than the density of the core 10128, approximately 30% greater than the density of the core 10128, approximately 40% greater than the density of the core 10128, approximately 50% greater than the density of the core 10128, approximately 60% greater than the density of the core 10128, approximately 70% greater than the density of the core 10128, approximately 80% greater than the density of the core 10128, approximately 90% greater than the density of the core 10128, and/or approximately 100% greater than the density of the core 10128, for example. The skin 10122 can comprise a density which is more than the density of the core 10128 and less than twice the density of the core 10128, for example. The skin 10122 can comprise a density which is over twice the density of the core 10128, for example. Further to the above, the skin 10122 and the core 10128 can be formed, or manufactured, simultaneously. For example, a fluid comprising any suitable material disclosed herein can be poured into a dish or mold and, while the fluid solidifies, the fluid can form a skin, or layer, which has a higher density than the remainder of the material. Optionally, multiple layers within a material can be formed by utilizing a process in which one or more subsequent layers of material are poured onto a previously cured layer. Two or more layers can be bonded to each other with an adhesive, for example. Two or more layers can be attached to each other by one or more fasteners and/or one or more mechanical interlocking features, for example. For example, adjacent layers can be connected together by one or more dovetail joints, for example. The skin can comprise a sealed surface which can prevent, or at least limit, the flow of fluid therethrough. The skin can comprise an open cell porous structure, for example.

Further to the above, the skin can be cut off of the tissue thickness compensator. The tissue thickness compensator can be cut from a larger block of material such that the tissue thickness compensator does not comprise a skin. For example, the tissue thickness compensator can be comprised of a homogenous, or at least substantially homogeneous, material, comprising large pores, for example.

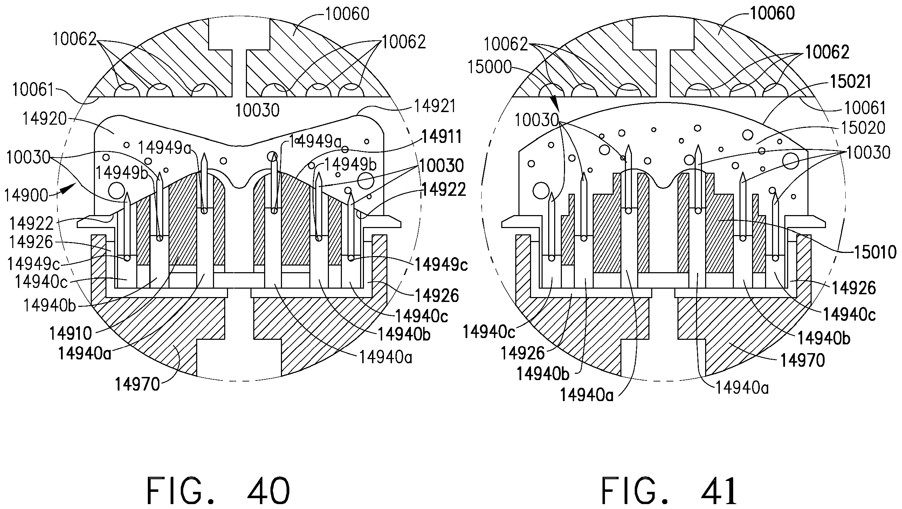

Referring now to FIG. 40, a staple cartridge, such as staple cartridge 14900, for example, can comprise a support portion 14910 and, in addition, a tissue thickness compensator 14920 positioned against the support portion 14910. Similar to the above, the support portion 14910 can comprise staple drivers which can be lifted upwardly by a staple-deploying sled in order to lift staples, such as staples 10030, for example, at least partially positioned within the support portion 14910 toward an anvil, such as anvil 10060, for example, positioned opposite the staple cartridge 14900. The support portion 14910 can comprise six rows of staple cavities, such as two outer rows of staple cavities, two inner rows of staple cavities, and two intermediate rows of staple cavities positioned intermediate the inner rows and the outer rows, for example, wherein the anvil 10060 can comprise six rows of forming pockets 10062 aligned, or at least substantially aligned, with the staple cavities. The inner rows of staple cavities can include staple drivers 14940a positioned therein, the intermediate rows of staple cavities can include staple drivers 14940b positioned therein, and the outer rows of staple cavities can include staple drivers 14940c positioned therein, wherein each of the staple drivers 14940a can include a cradle 14949a configured to support a staple 10030, wherein each of the staple drivers 14940b can include a cradle 14949b configured to support a staple 10030, and wherein each of the staple drivers 14940c can include a cradle 14949c configured to support a staple 10030. In their unfired positions, i.e., when the staple drivers 14940a-14940c are sitting on driver supports 14926 which extend underneath the support portion 14910, the cradles 14949a of the staple drivers 14940a can be positioned closer to the anvil 10060 than the cradles 14949b of the staple drivers 14940b and the cradles 14949c of the staple drivers 14940c. In such a position, a first forming distance can be defined between the cradles 14949a and the forming pockets 10062 positioned over the cradles 14949a, a second forming distance can be defined between the cradles 14949b and the forming pockets 10062 positioned over the cradles 14949b, and a third forming distance can be defined between the cradles 14949c and the forming pockets 10062 positioned over the cradles 14949c, wherein, the first forming distance can be shorter than the second forming distance and the second forming distance can be shorter than the third forming distance, for example. When the staple drivers 14940a-14940c are moved from their unfired positions (FIG. 40) to their fired positions, each staple driver 14940a-14940c can be moved upwardly an equal, or an at least substantially equal, distance toward the anvil 10060 by the staple-deploying sled such that the first drivers 14940a drive their respective staples 10030 to a first formed height, the second drivers 14940b drive their respective staples 10030 to a second formed height, and the third drivers 14940c drive their respective staples 10030 to a third formed height, wherein the first formed height can be shorter than the second formed height and the second formed height can be shorter than the third formed height, for example. Various alternatives are envisioned in which the first staple drivers 14940a are displaced upwardly a first distance, the second staple drivers 14940b are displaced upwardly a second distance, and the third staple drivers 14940c are displaced upwardly a third distance, wherein one or more of the first distance, the second distance, and the third distance can be different.

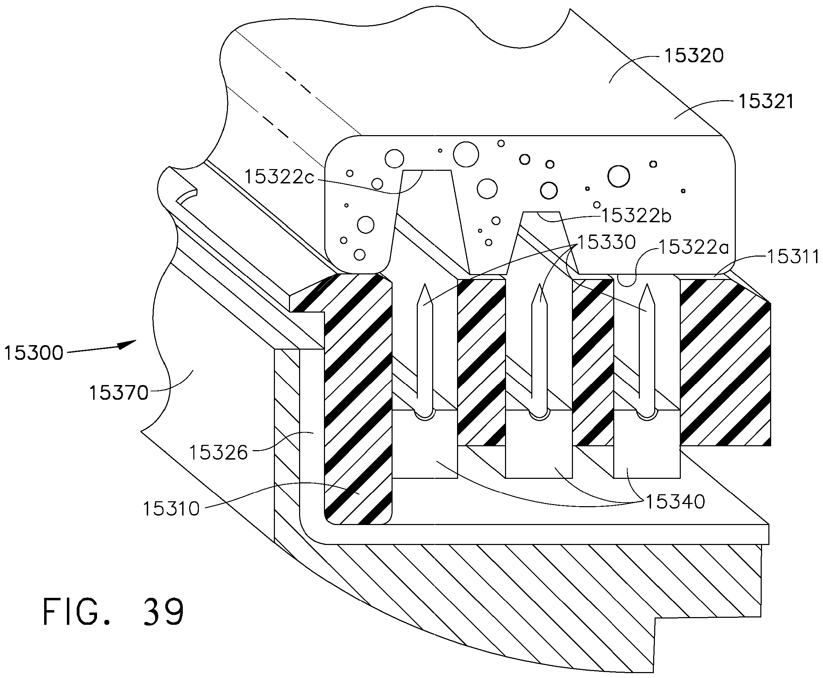

Referring again to FIG. 40, the deck surface 14911 of the support portion 14910 can vary in height with respect to the tissue-contacting surface 10061 of the anvil 10060. This height variation can occur laterally and, the height of the deck surface 14911 surrounding the inner rows of staple cavities can be higher than the deck surface 14911 surrounding the outer rows of staple cavities, for example. The bottom surface 14922 of the tissue thickness compensator 14920 can be configured to parallel, or at least substantially parallel, the deck surface 14911 of the support portion 14910. Further to the above, the tissue thickness compensator 14920 can also vary in thickness wherein, the top, or tissue-contacting, surface 14921 of the tissue thickness compensator 14920 can slope inwardly from the outside or lateral edges thereof. For example, as a result of the above, the tissue thickness compensator 14920 can be thinner in a region positioned over the inner rows of staple cavities and thicker in a region positioned over the outer rows of staple cavities, for example. Referring now to FIG. 41, the deck surface of a support portion 15010 can comprise a stepped deck surface, for example, wherein the highest steps of the stepped surface can surround the inner rows of staple cavities and the lowest steps of the stepped surface can surround the outer rows of staple cavities, for example. For example, steps having an intermediate height can surround the intermediate rows of staple cavities. A tissue thickness compensator, such as tissue thickness compensator 15020, for example, can comprise a bottom surface which can parallel and abut the deck surface of the support portion 15010. The top, or tissue-contacting, surface 15021 of the tissue thickness compensator can comprise an arcuate, parabolic, and/or curved surface, for example, which, for example, can extend from a first lateral side of the tissue thickness compensator 15020 to a second lateral side of the tissue thickness compensator 15020 with an apex aligned, or at least substantially aligned, with the center of the staple cartridge 15000, for example. Referring now to FIG. 39, a staple cartridge 15300, for example, can comprise a support portion 15310, a plurality of staple drivers 15340 movably positioned within staple cavities defined in the support portion 15310, and a tissue thickness compensator 15320 positioned above the deck surface 15311 of the support portion 15310. The staple cartridge 15300 can further comprise one or more bottom pan portions 15326 which can be attached to the support portion 15310 and extend around the bottom of the support portion 15310 and support the drivers 15340, and staples 15330, in their unfired positions. As a staple-deploying sled is advanced through the staple cartridge, the sled can also be supported by the bottom pan portions 15326 as the sled lifts the staple drivers 15340 and the staples 15330 upwardly through the tissue thickness compensator 15320. The tissue thickness compensator 15320 can comprise a first, or inner, portion 15322a positioned over an inner row of staple cavities, a second, or intermediate portion 15322b positioned over an intermediate row of staple cavities, and a third, or outer, portion 15322c positioned over a row of staple cavities, wherein the inner portion 15322a can be thicker than the intermediate portion 15322b and the intermediate portion 15322b can be thicker than the outer portion 15322c, for example. The tissue thickness compensator 15320 can comprise longitudinal channels, for example, defined therein which can create the thinner portions 15322b and 15322c of the tissue thickness compensator 15320. Alternatively, the longitudinal channels can be defined in the top surface and/or the bottom surface of a tissue thickness compensator. The top surface 15321 of the tissue thickness compensator 15320 can comprise a flat, or at least substantially flat, surface, for example.

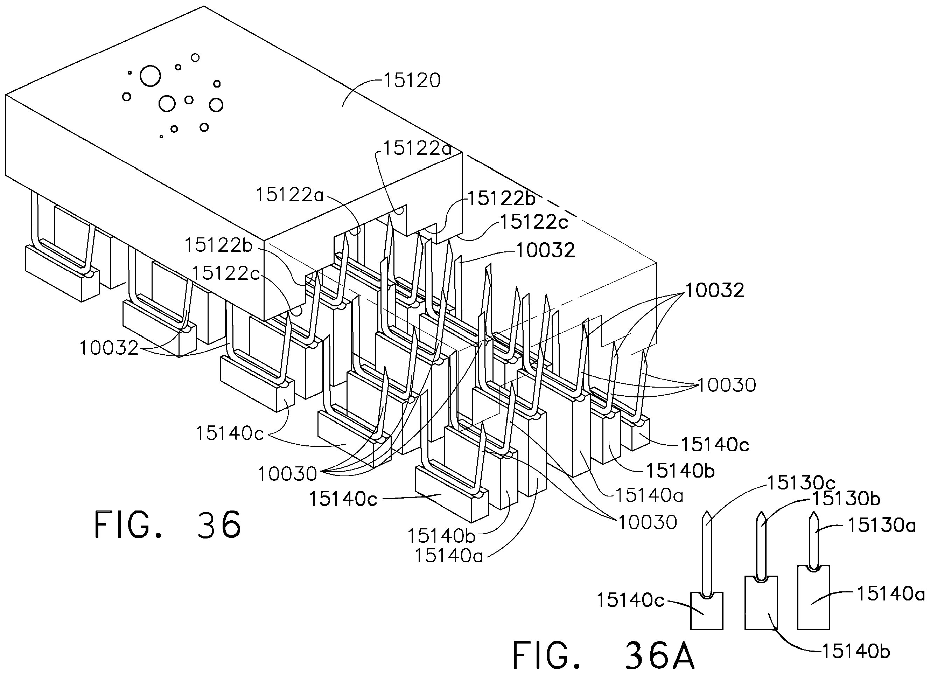

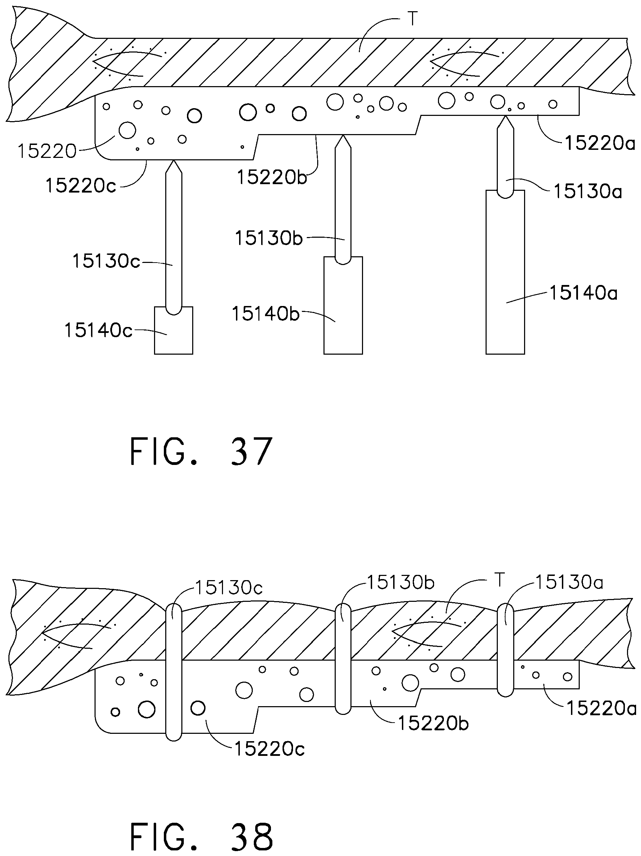

Referring now to FIG. 36, a staple cartridge can comprise a tissue thickness compensator, such as tissue thickness compensator 15120, for example, which can comprise a plurality of portions having different thicknesses. The tissue thickness compensator 15120 can comprise a first, or inner, portion 15122a which can have a first thickness, second, or intermediate, portions 15122b extending from the first portion 15122b which can each have a second thickness, and third, or outer, portions 15122c extending from the second portions 15122b which can each have a third thickness. For example, the third thickness can be thicker than the second thickness and the second thickness can be thicker than the first thickness, for example, although any suitable thicknesses could be utilized. The portions 15122a-15122c of the tissue thickness compensator 15120 can comprise steps having different thickness. Similar to the above, a staple cartridge can comprise several rows of staples 10030 and a plurality of staple drivers having different heights which can deform the staples 10030 to different formed heights. Also similar to the above, the staple cartridge can comprise first staple drivers 15140a which can drive the staples 10030 supported thereon to a first formed height, second staple drivers 15140b which can drive the staples 10030 supported thereon to a second formed height, and third staple drivers which can drive the staples 10030 supported thereon to a third formed height, wherein the first formed height can be shorter than the second formed height and the second formed height can be shorter than the third formed height, for example. Optionally, as illustrated in FIG. 36, each staple 10030 can comprise the same, or substantially the same, unformed, or unfired, height. Alternatively, referring now to FIG. 36A, the first drivers 15140a, the second drivers 15140b, and/or the third drivers 15140c can support staples having different unformed heights. For example, the first staple drivers 15140a can support staples 15130a having a first unformed height, the second staple drivers 15140b can support staples 15130b having a second unformed height, and the third staple drivers 15140c can support staples 15130c having a third unformed height, wherein the first unformed height can be shorter than the second unformed height and the second unformed height can be shorter than the third unformed height, for example. Referring again to FIG. 36A, the tips of the staples 15130a, 15130b, and/or 15130c can lie, or at least substantially lie, in the same plane while, alternatively, the tips of the staples 15130a, 15130b, and/or 15130c may not lie in same plane. Referring now to FIG. 37, a staple cartridge can include a tissue thickness compensator 15220 having a plurality of portions having different thickness which can be implanted against the tissue T by the staples 15130a, 15130b, and 15130c, as described above. Referring now to FIG. 38, the staples 15130a, 15130b, and/or 15130c can be deformed to different formed heights wherein the first staples 15130a can be formed to a first formed height, the second staples 15130b can be formed to a second formed height, and the third staples 15130c can be formed to a third formed height, and wherein the first formed height can be shorter than the second formed height and the second formed height can be shorter than the third formed height, for example. Alternatives are envisioned in which the staples 15130a, 15130b, and 15130c can be formed to any suitable formed heights and/or any relative formed heights.

In certain surgical applications, it may be advantageous to employ tissue thickness compensators with surgical stapling devices that are designed to cut and staple tissues having various tissue thickness profiles. Some devices employ different sizes of staples in side-by-side adjacent rows in the staple cartridge. Those staples may have different unformed heights or lengths so that, when driven through tissue into a confronting anvil, the final "formed height" of staples in one line of staples differ from the formed heights of the staples in another line. Other cartridge and anvil arrangements may employ stepped anvil decks (i.e., anvils that have staple forming surfaces that have stepped portions across their respective widths), stepped cartridge decks (i.e., cartridge decks that are not planar across their widths), different staple drivers and wedge sled arrangements, etc. for forming staples that have different formed or final heights. Examples of some surgical stapling instruments that employ some of these arrangements are disclosed in U.S. Patent Nos. 4,941,623; 5,0027,834, 7,669,746 and W/O 2003/094747A1.

When tissue thickness compensators that have a constant cross-sectional thickness are employed with such stapling devices, undesirable problems could occur. For example, in those arrangements where different heights of staples are arranged in side-by-side adjacent rows across the staple cartridge, if the tissue thickness compensator employed has a constant thickness, the shorter staples may have to be fired through too much foam (or other compensator material) and the longer staples may be fired through too little foam/material.

As will be discussed in further detail below, embodiments of the invention are directed to fabricating tissue thickness compensators that have non-uniform cross-sectional profiles or thicknesses that may, for example, be effectively employed with surgical stapling devices that are designed to form staples with different formed on final heights. Such tissue thickness compensators may be formed from a biocompatible or bioabsorbable polymer material such as, for example, foam, film fibrous non-woven PGA, PGA/PCL (poly(glycolic acid-co-caprolactone)), PLA/PCL (poly(lactic acid-co-polycaprolactone)), PGA/PLA (poly(glycolic acid-co-lactic acid)), PDS/PLA (poly(p-dioxanone-co-lactic acid), PGA/TMC(poly(glycolic acid-co-trimethylene carbonate)), PLLA/PCL, PGA/TMC, PDS, PEPBO or other absorbable polyurethane, polyester, polycarbonate, polycarbonate, polyorthoesters, polyanhydrides, polyesteramides, polyoxaesters, etc. that is suspended in an organic solvent material. Other suitable absorbable polymer materials such as those disclosed in U.S. Patent No. 6,333,029, entitled "Porous Tissue Scaffoldings For the Repair of Regeneration of Tissue", issued December 25, 2001, may also be used. Suitable solvents that should be generally suited as a starting place for selecting a solvent for the preferred absorbable aliphatic polyesters include but are not limited to solvents selected from a group consisting of formic acid, ethyl formate, acetic acid, hexafluoroisopropanol (HFIP), cyclic ethers (i.e. THF, DMF, and PDO), acetone, acetates of C2 to C5 alcohol (such as ethyl acetate and t-butylacetate), glyme (i.e. monoglyme, ethyl glyme, diglyme, ethyl diglyme, triglyme, butyl diglyme and tetraglyme) methylethyl ketone, dipropyleneglycol methyl ether, lactone-s (such as .gamma.-valerolactone, .delta.-valerolactone, .beta.-butyrolactone, .gamma.-butyrolactone) 1,4-dioxane, 1,3-dioxolane, 1,3-dioxolane-2-one (ethylene carbonate), dimethlycarbonate, benzene, toluene, benzyl alcohol, p-xylene, naphthalene, tetrahydrofuran, N-methyl pyrrolidone, dimethylformamide, chloroform, 1,2-dichloromethane, morpholine, dimethylsulfoxide, hexafluoroacetone sesquihydrate (HFAS), anisole and mixtures thereof. Among these solvents, the preferred solvent is 1,4-dioxane. A homogeneous solution 19010 of the polymer in the solvent is prepared using standard techniques. For example, the maximum concentrate of one of the above-mentioned polymer materials may be in the 10-20% polymer to 90-80% solvent. However, no matter how long the solution 19010 sits, the polymer does not precipitate back into solids. Other suitable solvents such as those disclosed in U.S. Patent No. 6,333,029 may be employed.

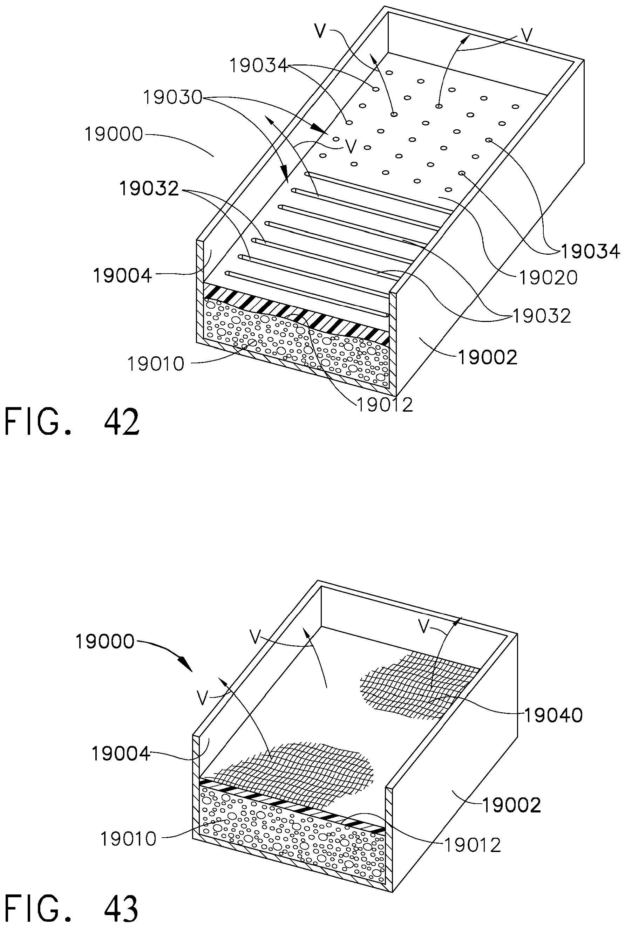

Tissue thickness compensators that may have different cross-sectional thicknesses and profiles may be formed. For example, FIG. 42 illustrates a mold arrangement 19000 that may be employed in connection with a lyophilization method for forming various tissue thickness compensator arrangements. In at least one form, the mold arrangement 19000 includes a mold 19002 that defines a mold cavity 19004 that is configured to form a tissue thickness compensator in a desired shape. The mold 19002 may be fabricated from, for example, metal such as aluminum, stainless steel, etc.

After the polymer solvent mixture is formed the mixture is then solidified. For a specific polymer-solvent system, the solidification point, the melt temperature and the apparent glass transition of the polymer-solvent system can be determined using standard differential scanning calorimetric (DSC) techniques. In theory, but in no way limiting the scope of the present invention, it is believed that as a polymer solvent system is cooled down an initial solidification occurs at about or below the freezing point of the solvent. This corresponds to the freezing of a substantial portion of the solvent in the system. The initial freezing appears as a first exothermic peak. A second freezing point occurs when the remaining solvent associated with the polymer solidifies. The second freezing point is marked by a second exothermic peak. The apparent Tg is the temperature at which the fully frozen system displays the first endothermic shift on reheating. An important parameter to control is the rate of freezing of the polymer-solvent system. The type of pore morphology that gets locked in during the freezing step is a function of the solution thermodynamics, freezing rate, temperature to which it is cooled, concentration of the solution, homogeneous or heterogeneous nucleation etc. Detailed description of these phase separation phenomenon can be found in the references provided herein ("Microcellular foams via phase separation" by A. T. Young, J. Vac. Sci. Technol. A 4(3), May/June 1986; and "Thermodynamics of Formation of Porous Polymeric Membrane from Solutions" by S. Matsuda, Polymer J. Vol. 23, No. 5, pp 435-444, 1991).

The polymer solution previously described can be solidified in a variety of manners such as placing or injecting the solution in a mold cavity 19004 and cooling the mold 19002 in an appropriate bath or on a refrigerated shelf. The mold 19002 may be supported within a pressure vessel (not shown) wherein the ambient pressure may be controlled. After the solution 19010 has been frozen, the ambient pressure is reduced. For example, the pressure may be reduced to a few millibars. In addition, the temperature of the solution 19010 is increased. This may be accomplished by increasing the temperature of the mold 19002. Further details regarding these processes may be found in U.S. Patent No. 6,333,029.

A biocompatible/bioabsorbable film material 19020 can be employed. The film material may be made from the same monomer or copolymer as the polymer comprising the foam or it may comprise a different material selected from the list of materials provided above. To enable the liquid solution 19010 to sublimate gas vapor, the film 19020 must be air permeable and/or it must contain openings through which the gas vapor can pass. As the pressure is reduced and the temperature is increased, the solvent sublimates through the air permeable film or the openings therein. Once the solvent vapor has sublimated, the solid foam tissue thickness compensator may be removed from the mold for use with a surgical stapling device.

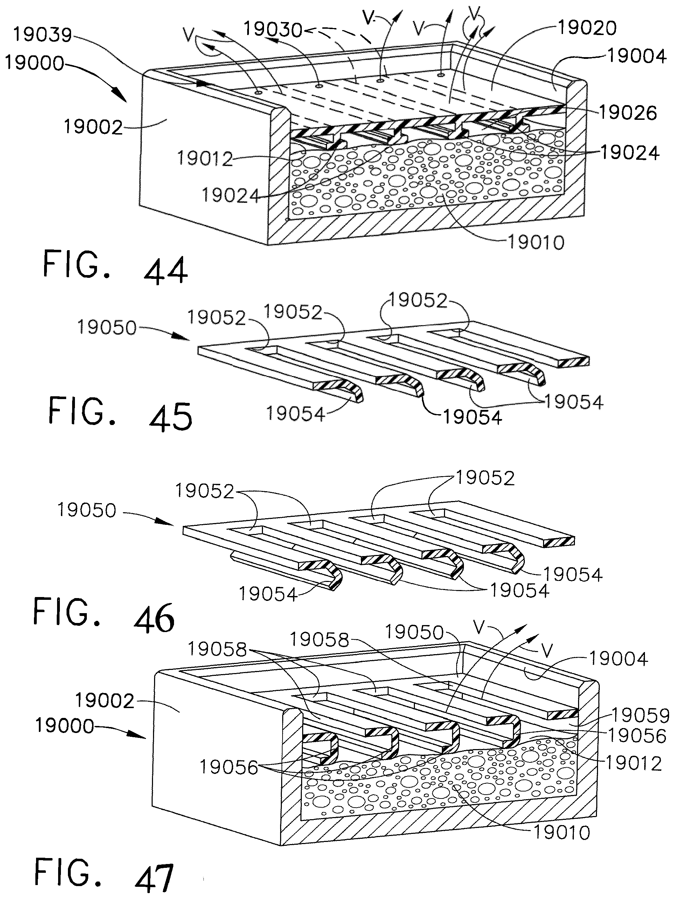

FIG. 42 illustrates a layer of film 19020 which is not in and of itself air permeable. Thus, openings 19030 are provided through the film 19020. As can be seen in FIG. 42 for example, the film 19020 has been placed onto an exposed surface 19012 of the foam solution 19010 and the vapor (represented by arrows "V" in FIG. 42) is permitted to sublime or pass through the openings 19030. A variety of different shapes, numbers and densities of openings 19030 may be employed. As depicted, two different opening configurations are shown. For example, the openings 19030 may comprise elongated slots 19032 and or round holes 19034. However, other shapes, numbers, and sizes of openings can also be successfully employed. For example, openings 19030 may also comprise perforations, channels, slits, etc. As the vapor "V" sublimes through the openings 19030, the solution 19010 solidifies into a foam with open cells where the solvent vapors previously resided. Additional vent openings 19039 may be provided through the film 19020 at the ends of the open channels between the film and the liquid material 19010 to further facilitate degassing. See FIG. 44. After the solvent vapor has sublimated, the foam tissue thickness compensator may be removed from the mold 19002 and used in any of the above described manners.

FIG. 43 illustrates use of an alternative film 19040 which may comprise biocompatible material such as for example, bioabsorbable thin punctured mesh. The small round or square holes, for example, allow the solvent vapor to pass therethrough just as the various openings did in the earlier embodiment. The vapors V are able to sublime through such material. FIG. 44 illustrates use of film 19020 of the types described above that has a series of standoff structures 19024 formed thereon or otherwise attached thereto to retain the film 19020 in a spaced-apart relationship relative to the exposed surface 19012 and create air space 19026 therebetween which serves to promote sublimation of the vapors V. In addition, a series of perforations or openings 19030 may be provided through the film 19020 through which the vapors "V" may pass. As illustrated, the stand off structures 19024 comprise T-shaped members. Other shapes of standoff structures may be employed. For example, in other cases, stand off "pillows" may be integrally formed into the film 19020 to retain the film 19020 in a spaced- apart relationship relative to the exposed surface 19012. Such arrangement serves to minimize the contact points between the film and the foam.

FIGS. 45-47 illustrate the formation and use of a film 19050 wherein a plurality of cuts 19052 are formed in the film 19050 to form flap areas 19054. Thereafter, the flap areas 19054 are folded as shown in FIGS. 45 and 46 such that the flaps 19054 form standoffs 19056 that each have a cross-sectional shape that is roughly C-shaped. Such arrangement forms openings 19058 in the film 19050 through which the vapors V may sublime. Once the liquid solution 19010 has been placed into the cavity 19004, the film 19050 is placed onto the exposed surface 19012 such that the standoffs 19056 retain the film 19050 spaced above the exposed surface 19012 to define an air space 19059 therebetween.

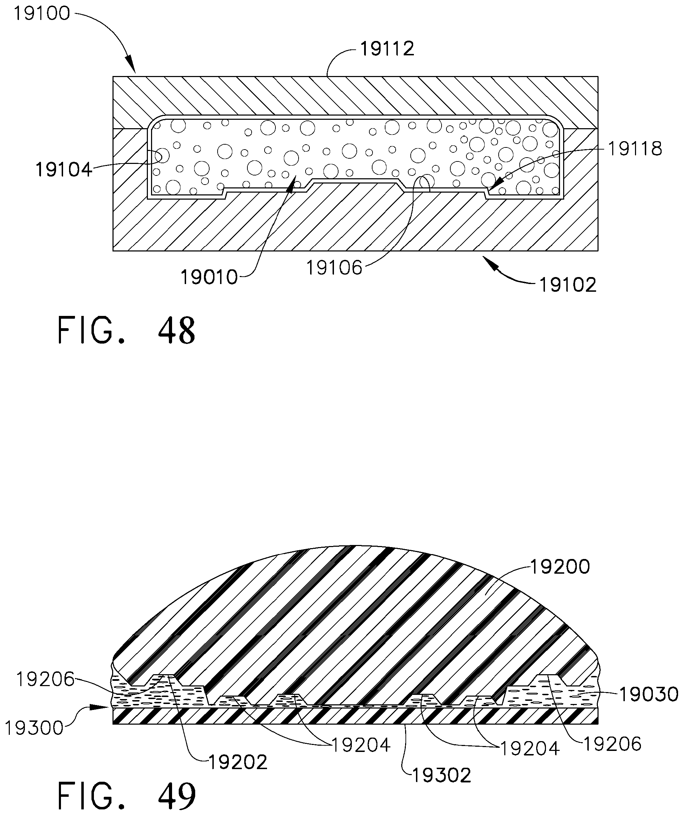

FIG. 48 depicts a mold arrangement 19100 that may be employed in connection with a lyophilization method for forming various forms of tissue thickness compensator arrangements of the types disclosed above. In at least one form, the mold arrangement 19100 includes a mold 19102 that defines a mold cavity 19104 that is configured to form a tissue thickness compensator having a desired shaped. As illustrated, for example, the mold cavity 19104 includes a bottom surface 19106 that has a predetermined profile 19108. The mold 19102 may further have a lid 19112 thereon to further define the final shape of the tissue thickness compensator formed in the mold 19102. The lid 19112 has openings (not shown) therethrough to permit passage of the vapors. The liquid solution 19010 is introduced into the mold cavity 19104 and the solvent is permitted to sublime.

FIG. 49 illustrates in cross-section an anvil 19200 of a surgical stapling instrument that has an irregular (no-planar or stepped) staple-forming surface 19202 thereon. As can be seen in that Figure, the staple forming surface 19202 defines staple forming pockets 19204 and 19206 that have different sizes and shapes. The surface 19202 additionally is stepped across-its width. That is, various lines of staple pockets lie on different planes. The above-described molding process may be employed to form a tissue thickness compensator 19300 that substantially conforms to or substantially matches the profile of the staple forming surface 19202. For example, the bottom surface of the mold is provided with a profile that matches the staple forming surface 19202. In FIG. 49, a tissue thickness compensator 19300 was formed with a film 19302.

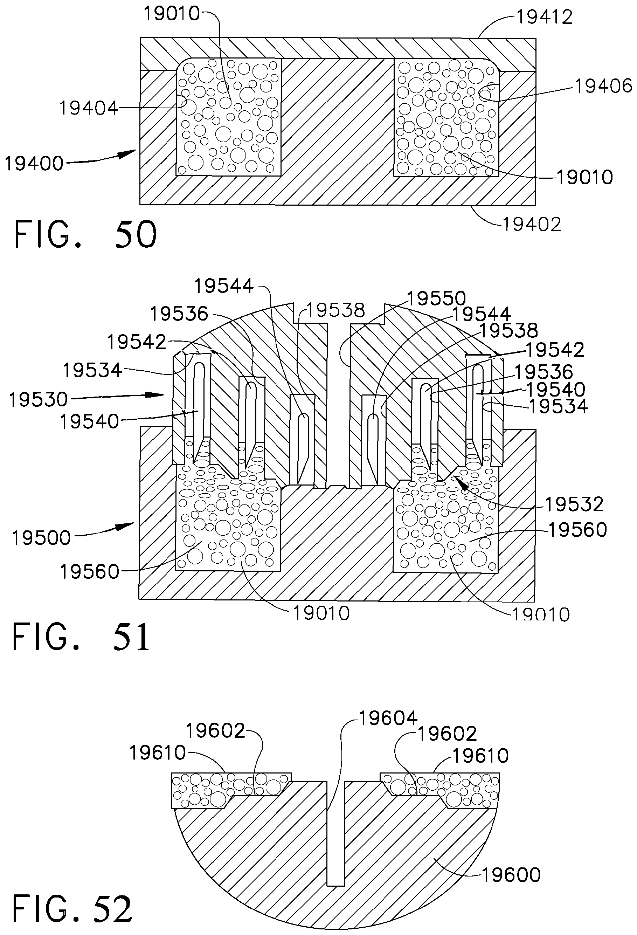

FIG. 50 depicts a mold arrangement 19400 that may be employed in connection with a lyophilization method for forming various forms of tissue thickness compensator arrangements of the types disclosed above. In at least one form, the mold arrangement 19400 includes a mold 19402 that defines two mold cavities 19404, 19406 that are configured to form two separated tissue thickness compensators, each having a desired shaped. The mold 19402 may further have a lid 19412 thereon to further define the final shape of the tissue thickness compensators formed in the mold 19402. The lid 19112 has openings (not shown) therethrough to permit passage of the vapors. The liquid solution 19010 is introduced into each of the mold cavities 19404, 19408 and is permitted to cure.

FIG. 51 depicts a mold arrangement 19500 that may be employed in connection with a lyophilization method for forming various forms of tissue thickness compensator arrangements of the types disclosed above on the deck of a staple cartridge 19530. As depicted, the staple cartridge 19530 has an irregular or non-planar or "stepped" staple deck 19532. Various staple cartridges are described in, for example, U.S. Patent No. 7,669,746, issued March 2, 2010. As can be seen in FIG. 51, a series of staple openings 19534, 1536, 19538 are provided through the staple deck 19532. Each of the staple cavities 19534, 19536, 19538 support one or more surgical staples 19540, 19542, 19544, respectively, therein as is generally shown in FIG. 51. As can be seen in that Figure, the staples in this cartridge embodiment have different unformed lengths or heights. That is, as illustrated, unformed staples 19540 are longer than unformed staples 19542 which are longer than unformed staples 19544.

Still referring to FIG. 51, the mold arrangement 19500 includes a mold 19502 that defines two mold cavities 19504, 19506 that are configured to form two separated tissue thickness compensator segments corresponding to the staple cavities 19534, 19536 on each side of a knife slot 19550 in the staple cartridge 19530. The liquid solution 19010 is introduced into each of the mold cavities 19504, 19506 and then frozen as described above. The surgical staple cartridge 19530 is then supported on the mold 19502 as shown in FIG. 51. The pressure is reduced and the temperature of the solution is increased causing the solvent vapor to sublime. As the solvent vapor sublimes, the remaining polymer material expands/enters into the staple cavities 19534, 19536 as shown. Once the solvent vapor has sublimated, the staple cartridge 19530 is removed from the mold 19502 with the tissue thickness compensators 19560 formed thereon. In such arrangement, the tissue thickness compensators 19560 are formed to coincide with less than all of the staple cavities extending laterally across the staple cartridge deck. The tissue thickness compensators 19560 extend into the staple cavities and are attached to the tips of the staples housed therein. Such manufacturing method provides a way of achieving focused staple line reinforcement with tissue thickness compensators. FIG. 52 illustrates a surgical stapler anvil 19600 with tissue thickness compensators 195610 formed on the staple forming surface 19602 on each side of a central knife slot 19604 using the above described molding methods.

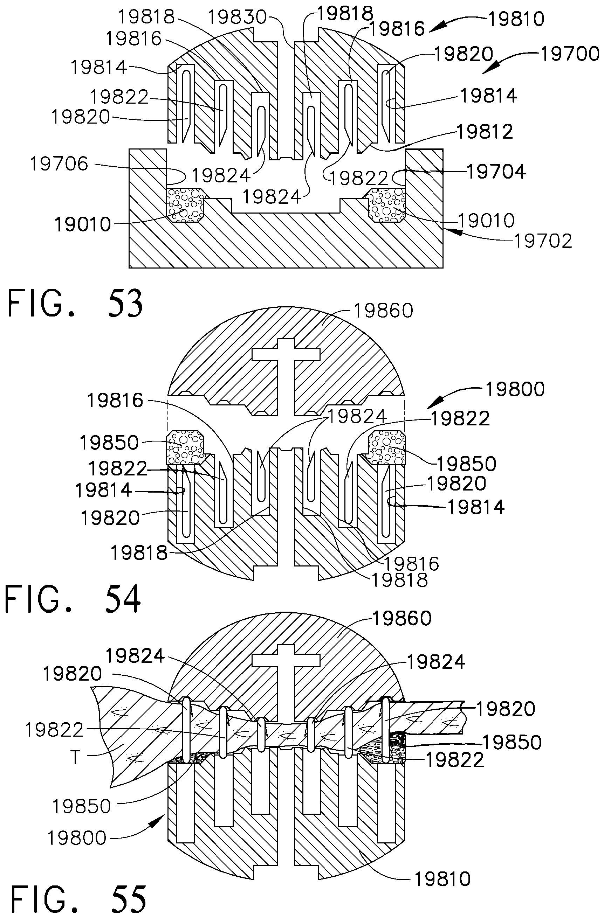

FIG. 53 depicts a mold arrangement 19700 that may be employed in connection with a lyophilization method for forming various forms of tissue thickness compensator arrangements of the types disclosed above on the deck of a staple cartridge 19810 of a surgical stapling instrument 19800 depicted in FIG. 54. As can be seen in FIGS. 53 and 54, the staple cartridge 19810 has an irregular or non-planar staple deck 19812. The cartridge 19810 includes, for example, a series of staple openings 19814, 19816, 19818 are provided through the staple deck 19812. Each of the staple cavities 19814, 19816, 19818 support one or more surgical staples 19820, 19822, 19824, respectively, therein as is generally shown in FIGS. 53 and 54.

Still referring to FIG. 53, the mold arrangement 19700 includes a mold 19702 that defines two mold cavities 19704, 19706 that are configured to form two separated tissue thickness compensator segments19850 corresponding to the staple cavities 19814 on each side of a knife slot 19830 in the staple cartridge 19810. The liquid solution 19010 is introduced into each of the mold cavities 19704, 19706 and then frozen. The surgical staple cartridge 19810 is introduced onto the mold 19702 as shown in FIG. 53 and the ambient pressure is reduced and the temperature is increased to cause the solvent vapor to sublimate. Once the solvent vapor has sublimated leaving the polymer foam, the staple cartridge 19810 is removed from the mold 19702 with the tissue thickness compensators 19850 formed thereon. See Fig. 54. FIG. 55 illustrates use of the surgical stapling instrument 19800 to staple through tissue T having a varying thickness. As can be seen in that Figure, the tissue thickness compensators 19850 serve to compensate for staples that are may otherwise be too long for the thickness of the tissue being stapled.

Further to the above, a tissue thickness compensator can be comprised of a biocompatible material. The biocompatible material, such as, a foam, may comprise tackifiers, surfactants, fillers, cross-linkers, pigments, dyes, antioxidants and other stabilizers and/or combinations thereof to provide desired properties to the material. A biocompatible foam may comprise a surfactant. The surfactant may be applied to the surface of the material and/or dispersed within the material. Without wishing to be bound to any particular theory, the surfactant applied to the biocompatible material may reduce the surface tension of the fluids contacting the material. For example, the surfactant may reduce the surface tension of water contacting the material to accelerate the penetration of water into the material. The water may act as a catalyst. The surfactant may increase the hydrophilicity of the material.

The surfactant may comprise an anionic surfactant, a cationic surfactant, and/or a nonionic surfactant. Examples surfactants include, but are not limited to polyacrylic acid, methalose, methyl cellulose, ethyl cellulose, propyl cellulose, hydroxy ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, and polyoxamers, and combinations thereof. The surfactant may comprise a copolymer of polyethylene glycol and polypropylene glycol. The surfactant may comprise a phospholipid surfactant. The phospholipid surfactant may provide antibacterial stabilizing properties and/or disperse other materials in the biocompatible material.

The tissue thickness compensator may comprise at least one medicament. The tissue thickness compensator may comprise one or more of the natural materials, non-synthetic materials, and/or synthetic materials described herein. The tissue thickness compensator may comprise a biocompatible foam comprising gelatin, collagen, hyaluronic acid, oxidized regenerated cellulose, polyglycolic acid, polycaprolactone, polyactic acid, polydioxanone, polyhydroxyalkanoate, poliglecaprone, and combinations thereof. The tissue thickness compensator may comprise a film comprising the at least one medicament. The tissue thickness compensator may comprise a biodegradable film comprising the at least one medicament. The medicament may comprise a liquid, gel, and/or powder. The medicaments may comprise anticancer agents, such as, for example, cisplatin, mitomycin, and/or adriamycin.

The tissue thickness compensator may comprise a biodegradable material to provide controlled elution of the at least one medicament as the biodegradable material degrades. The biodegradable material may degrade may decompose, or loses structural integrity, when the biodegradable material contacts an activator, such as, for example an activator fluid. The activator fluid may comprise saline or any other electrolyte solution, for example. The biodegradable material may contact the activator fluid by conventional techniques, including, but not limited to spraying, dipping, and/or brushing. In use, for example, a surgeon may dip an end effector and/or a staple cartridge comprising the tissue thickness compensator comprising the at least one medicament into an activator fluid comprising a salt solution, such as sodium chloride, calcium chloride, and/or potassium chloride. The tissue thickness compensator may release the medicament as the tissue thickness compensator degrades. The elution of the medicament from the tissue thickness compensator may be characterized by a rapid initial elution rate and a slower sustained elution rate.

According to the invention, a tissue thickness compensator, for example, can be comprised of a biocompatible material which may comprise an oxidizing agent. The oxidizing agent may an organic peroxide and/or an inorganic peroxide. Examples of oxidizing agents may include, but are not limited to, hydrogen peroxide, urea peroxide, calcium peroxide, and magnesium peroxide, and sodium percarbonate. The oxidizing agent may comprise peroxygen-based oxidizing agents and hypohalite-based oxidizing agents, such as, for example, hydrogen peroxide, hypochlorous acid, hypochlorites, hypocodites, and percarbonates. The oxidizing agent may comprise alkali metal chlorites, hypochlorites and perborates, such as, for example, sodium chlorite, sodium hypochlorite and sodium perborate. The oxidizing agent may comprise vanadate. The oxidizing agent may comprise ascorbic acid. The oxidizing agent may comprise an active oxygen generator. According to the invention, a tissue scaffold may comprise the biocompatible material comprising an oxidizing agent.

The biocompatible material may comprise a liquid, gel, and/or powder. The oxidizing agent may comprise microparticles and/or nanoparticles, for example. For example, the oxidizing agent may be milled into microparticles and/or nanoparticles. The oxidizing agent may be incorporated into the biocompatible material by suspending the oxidizing agent in a polymer solution. The oxidizing agent may be incorporated into the biocompatible material during the lyophylization process. After lyophylization, the oxidizing agent may be attached to the cell walls of the biocompatible material to interact with the tissue upon contact. The oxidizing agent may not be chemically bonded to the biocompatible material. A percarbonate dry power may be embedded within a biocompatible foam to provide a prolonged biological effect by the slow release of oxygen. A percarbonate dry power may be embedded within a polymeric fiber in a non-woven structure to provide a prolonged biological effect by the slow release of oxygen. The biocompatible material may comprise an oxidizing agent and a medicament, such as, for example, doxycycline and ascorbic acid.

The biocompatible material may comprise a rapid release oxidizing agent and/or a slower sustained release oxidizing agent. The elution of the oxidizing agent from the biocompatible material may be characterized by a rapid initial elution rate and a slower sustained elution rate. The oxidizing agent may generate oxygen when the oxidizing agent contacts bodily fluid, such as, for example, water. Examples of bodily fluids may include, but are not limited to, blood, plasma, peritoneal fluid, cerebral spinal fluid, urine, lymph fluid, synovial fluid, vitreous fluid, saliva, gastrointestinal luminal contents, and/or bile. Without wishing to be bound to any particular theory, the oxidizing agent may reduce cell death, enhance tissue viability and/or maintain the mechanical strength of the tissue to tissue that may be damaged during cutting and/or stapling.

The biocompatible material may comprise at least one microparticle and/or nanoparticle. The biocompatible material may comprise one or more of the natural materials, non-synthetic materials, and synthetic materials described herein. The biocompatible material may comprise particles having a mean diameter of about 10 nm to about 100 nm and/or about 10 µm to about 100 µm, such as, for example, 45-50 nm and/or 45-50 µm. The biocompatible material may comprise biocompatible foam comprising at least one microparticle and/or nanoparticle embedded therein. The microparticle and/or nanoparticle may not be chemically bonded to the biocompatible material. The microparticle and/or nanoparticle may provide controlled release of the medicament. The microparticle and/or nanoparticle may comprise at least one medicament. The microparticle and/or nanoparticle may comprise a hemostatic agent, an anti-microbial agent, and/or an oxidizing agent, for example. The tissue thickness compensator may comprise a biocompatible foam comprising an hemostatic agent comprising oxidized regenerated cellulose, an anti-microbial agent comprising doxycline and/or Gentamicin, and/or an oxidizing agent comprising a percarbant. The microparticle and/or nanoparticle may provide controlled release of the medicament up to three days, for example.

The microparticle and/or nanoparticle may be embedded in the biocompatible material during a manufacturing process. For example, a biocompatible polymer, such as, for example, a PGA/PCL, may contact a solvent, such as, for example, dioxane to form a mixture. The biocompatible polymer may be ground to form particles. Dry particles, with or without ORC particles, may be contacted with the mixture to form a suspension. The suspension may be lyophilized to form a biocompatible foam comprising PGA/PCL having dry particles and/or ORC particles embedded therein.

The tissue thickness compensators or layers disclosed herein can be comprised of an absorbable polymer, for example. A tissue thickness compensator can be comprised of foam, film, fibrous woven, fibrous non-woven PGA, PGA/PCL (Poly( glycolic acid-co-caprolactone)), PLA/PCL (Poly( lactic acid-co- polycaprolactone)), PLLA/PCL, PGA/TMC (Poly(glycolic acid-co-trimethylene carbonate)), PDS, PEPBO or other absorbable polyurethane, polyester, polycarbonate, Polyorthoesters, Polyanhydrides, Polyesteramides, and/or Polyoxaesters, for example. According to the invention, a tissue thickness compensator can be comprised of PGA/PLA (Poly(glycolic acid-co-lactic acid)) and/or PDS/PLA (Poly(p-dioxanone-co-lactic acid)), for example. According to the invention, a tissue thickness compensator can be comprised of an organic material, for example. A tissue thickness compensator can be comprised of Carboxymethyl Cellulose, Sodium Alginate, Cross-linked Hyaluronic Acid, and/or Oxidized regenerated cellulose, for example. According to the invention, a tissue thickness compensator can comprise a durometer in the 3-7 Shore A (30-50 Shore OO) ranges with a maximum stiffness of 15 Shore A (65 Shore OO), for example. A tissue thickness compensator can undergo 40% compression under 3 lbf load, 60% compression under 6 lbf load, and/or 80% compression under 20 lbf load, for example. One or more gasses, such as air, nitrogen, carbon dioxide, and/or oxygen, for example, can be bubbled through and/or contained within the tissue thickness compensator. A tissue thickness compensator can comprise beads therein which comprise between approximately 50% and approximately 75% of the material stiffness comprising the tissue thickness compensator.

According to the invention, a tissue thickness compensator can comprise hyaluronic acid, nutrients, fibrin, thrombin, platelet rich plasma, Sulfasalazine (Azulfidine^{®} - 5ASA+Sulfapyridine diazo bond))- prodrug - colonic bacterial (Azoreductase), Mesalamine (5ASA with different prodrug configurations for delayed release), asacol^{®} (5ASA + Eudragit-S coated -pH > 7 (coating dissolution)), Pentasa^{®} (5ASA + ethylcellulose coated - time/pH dependent slow release), Mesasal^{®} (5ASA + Eudragit-L coated - pH > 6), Olsalazine (5ASA + 5ASA - colonic bacterial (Azoreductase)), Balsalazide (5ASA + 4Aminobenzoyl-B-alanine) - colonic bacterial (Azoreductase)), Granulated mesalamine, Lialda (delay and SR formulation of mesalamine), HMPL-004 (herbal mixture that may inhibit TNF-alpha, interleukin-1 beta, and nuclear-kappa B activation), CCX282-B (oral chemokine receptor antagonist that interferes with trafficking of T lymphocytes into the intestinal mucosa), Rifaximin (nonabsorbable broad-spectrum antibiotic ), Infliximab, murine chymieric (monoclonal antibody directed against TNF-alpha-approved for reducing signs/symptoms and maintaining clinical remission in adult/pediatric patients with moderate/severe luminal and fistulizing Crohn's disease who have had inadequate response to conventional therapy), adalimumab, Total Human IgG1 (anti-TNF-alpha monoclonal antibody - approved for reducing signs/symptoms of Crohn's disease, and for the induction and maintenance of clinical remission in adult patients with moderate/severe active Crohn's disease with inadequate response to conventional therapies, or who become intolerant to Infliximab), Certolizumab pegoll, humanized anti-TNF FAB' (monoclonal antibody fragment linked to polyethylene glycol - approved for reducing signs/symptoms of Crohn's disease and for the induction and maintenance of response in adult patients w/ moderate/severe disease with inadequate response to conventional therapies), Natalizumab, First non-TNF-alpha inhibitor (biologic compound approved for Crohn's disease), Humanized monoclonal IgG4 antibody (directed against alpha-4 integrin - FDA approved for inducing and maintaining clinical response and remission in patients with moderate/severe disease with evidence of inflammation and who have had inadequate response to or are unable to tolerate conventional Crohn's therapies and inhibitors of TNF-alpha), concomitant Immunomodulators potentially given with Infliximab, azathioprine 6-Mercaptopurine (purine synthesis inhibitor - prodrug), Methotrexate (binds dihydrofolate reductase (DHFR) enzyme that participates in tetrahydrofolate synthesis, inhibits all purine synthesis), allopurinol and Thioprine therapy, PPI, H2 for acid suppression to protect the healing line, C-Diff - Flagyl, Vancomycin (fecal translocation treatment; probiotics; repopulation of normal endoluminal flora), and/or Rifaximin (treatment of bacterial overgrowth (notably hepatic encephalopathy); not absorbed in GI tract with action on intraluminal bacteria), for example.

As described herein, a tissue thickness compensator can compensate for variations in the thickness of tissue that is captured within the staples ejected from a staple cartridge and/or contained within a staple line, for example. Stated another way, certain staples within a staple line can capture thick portions of the tissue while other staples within the staple line can capture thin portions of the tissue. In such circumstances, the tissue thickness compensator can assume different heights or thicknesses within the staples and apply a compressive force to the tissue captured within the staples regardless of whether the captured tissue is thick or thin. According to the invention, a tissue thickness compensator can compensate for variations in the hardness of the tissue. For instance, certain staples within a staple line can capture highly compressible portions of the tissue while other staples within the staple line can capture portions of the tissue which are less compressible. In such circumstances, the tissue thickness compensator can be configured to assume a smaller height within the staples that have captured tissue having a lower compressibility, or higher hardness, and, correspondingly, a larger height within the staples that have captured tissue having a higher compressibility, or lower hardness, for example. In any event, a tissue thickness compensator, regardless of whether it compensates for variations in tissue thickness and/or variations in tissue hardness, for example, can be referred to as a 'tissue compensator' and/or as a 'compensator', for example.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

## Claims

1. A surgical stapling device, comprising:
a surgical staple cartridge (15300, 14900) including a deck surface (15311, 14911) and housing a plurality of surgical staples (10030, 15130, 15330); and
an anvil (10060, 19200, 19600) including a staple-forming surface and being movably supported relative to the surgical staple cartridge such that the staple-forming surface may be positioned in confronting relationship relative to the staple cartridge deck surface (15311, 14911);
wherein the stapling device is configured to form the plurality of staples housed in the staple cartridge (15300, 14900) against the staple-forming surface of the anvil (10060, 19200, 19600) in confronting relationship relative to the staple deck surface (15311, 14911) such that the staples are formed with different formed heights;
**characterized by** a tissue thickness compensator (15120, 15220, 15320, 14920, 15020, 19010, 19030, 19610) positioned between the staple forming surface of the anvil and the staple cartridge deck surface and including a non-uniform thickness measured from the staple-forming surface of the anvil to the staple cartridge deck surface.

2. The surgical stapling device of claim 1 wherein the plurality of surgical staples comprises a plurality of first surgical staples (15130a, 19540) having a first unformed height and a plurality of second surgical staples (15130b, 19542) having a second unformed height that is different from the first unformed height.

3. The surgical stapling device of claim 2 further comprising a plurality of third surgical staples (15130c, 19544) having a third unformed height that differs from the first and second unformed heights.

4. The surgical stapling device of claim 3 wherein the surgical staple cartridge further comprises an elongate slot (19550) extending through a portion thereof for accommodating a tissue cutting member and wherein the first surgical staples (19540) are arranged in first lines of surgical staples located on each lateral side of the slot and the second surgical staples (19542) are arranged in second lines of surgical staples located on each lateral side of the slot and wherein each third surgical staple (19544) is arranged in third lines of surgical staples located on each lateral side of the slot.

5. The surgical stapling device of any preceding claim wherein the deck surface (14911) of the surgical staple cartridge is non-planar and wherein the tissue thickness compensator (14920) includes a body portion including a surface profile that substantially matches the non-planar surface of the deck surface.

6. The surgical stapling device of claim 5 wherein the body portion is fabricated from biocompatible foam material.

7. The surgical stapling device of claim 5 or claim 6 further comprising an air permeable biocompatible film (19020) attached to the body portion.

8. The surgical stapling device of any preceding claim wherein the staple-forming surface of the anvil is non-planar and wherein the tissue thickness compensator (19610) includes a body portion including a surface profile that substantially matches the non-planar staple-forming surface.

## Patentansprüche

1. Chirurgische Klammervorrichtung, umfassend:
ein chirurgisches Klammermagazin (15300, 14900), das eine Deckfläche (15311, 14911) aufweist und eine Mehrzahl chirurgischer Klammern (10030, 15130, 15330) beherbergt; und
ein Widerlager (10060, 19200, 19600), das eine Klammerformungsfläche aufweist und relativ zu dem chirurgischen Klammermagazin derart beweglich gelagert ist, dass die Klammerformungsfläche in gegenüberliegender Beziehung relativ zu der Klammermagazindeckfläche (15311, 14911) positioniert werden kann;
wobei die Klammervorrichtung dazu ausgelegt ist, die Mehrzahl von in dem Klammermagazin (15300, 14900) beherbergten Klammern gegen die Klammerformungsfläche des Widerlagers (10060, 19200, 19600) in gegenüberliegender Beziehung relativ zu der Klammerdeckfläche (15311, 14911) derart zu formen, dass die Klammern mit unterschiedlichen geformten Höhen geformt werden;
**gekennzeichnet durch** einen Gewebedickenkompensator (15120, 15220, 15320, 14920, 15020, 19010, 19030, 19610), der zwischen der Klammerformungsfläche des Widerlagers und der Klammermagazindeckfläche positioniert ist und, gemessen von der Klammerformungsfläche des Widerlagers zu der Klammermagazindeckfläche, eine nicht einheitliche Dicke aufweist.

2. Chirurgische Klammervorrichtung nach Anspruch 1, wobei die Mehrzahl von chirurgischen Klammern eine Mehrzahl von ersten chirurgischen Klammern (15130a, 19540), die eine erste ungeformte Höhe aufweisen, und eine Mehrzahl von zweiten chirurgischen Klammern (15130b, 19542), die eine zweite ungeformte Höhe aufweisen, die sich von der ersten ungeformten Höhe unterscheidet, umfasst.

3. Chirurgische Klammervorrichtung nach Anspruch 2, ferner umfassend eine Mehrzahl von dritten chirurgischen Klammern (15130c, 19544), die eine dritte ungeformte Höhe aufweisen, die sich von der ersten und der zweiten ungeformten Höhe unterscheidet.

4. Chirurgische Klammervorrichtung nach Anspruch 3, wobei das chirurgische Klammermagazin ferner einen länglichen Schlitz (19550) umfasst, der sich durch einen Abschnitt davon zum Aufnehmen eines Gewebeschneidelements erstreckt, und wobei die ersten chirurgischen Klammern (19540) in ersten Reihen von chirurgischen Klammern angeordnet sind, die auf jeder lateralen Seite des Schlitzes angeordnet sind, und die zweiten chirurgischen Klammern (19542) in zweiten Reihen von chirurgischen Klammern angeordnet sind, die auf jeder lateralen Seite des Schlitzes angeordnet sind, und wobei jede dritte chirurgische Klammer (19544) in dritten Reihen von chirurgischen Klammern angeordnet ist, die auf jeder lateralen Seite des Schlitzes angeordnet sind.

5. Chirurgische Klammervorrichtung nach einem der vorhergehenden Ansprüche, wobei die Deckfläche (14911) des chirurgischen Klammermagazins nicht ebenflächig ist und wobei der Gewebedickenkompensator (14920) einen Körperabschnitt aufweist, der ein Oberflächenprofil aufweist, das im Wesentlichen mit der nicht ebenflächigen Oberfläche der Deckfläche übereinstimmt.

6. Chirurgische Klammervorrichtung nach Anspruch 5, wobei der Körperabschnitt aus biokompatiblem Schaummaterial hergestellt ist.

7. Chirurgische Klammervorrichtung nach Anspruch 5 oder Anspruch 6, ferner umfassend einen luftdurchlässigen biokompatiblen Film (19020), der an dem Körperabschnitt angebracht ist.

8. Chirurgische Klammervorrichtung nach einem der vorhergehenden Ansprüche, wobei die Klammerformungsfläche des Widerlagers nicht ebenflächig ist und wobei der Gewebedickenkompensator (19610) einen Körperabschnitt aufweist, der ein Oberflächenprofil aufweist, das im Wesentlichen mit der nicht ebenflächigen Klammerformungsfläche übereinstimmt.

## Revendications

1. Dispositif d'agrafage chirurgical, comprenant :
une cartouche d'agrafes chirurgicales (15300, 14900) comprenant une surface de plateau (15311, 14911) et logeant une pluralité d'agrafes chirurgicales (10030, 15130, 15330) ; et
une enclume (10060, 19200, 19600) comprenant une surface de formation d'agrafe et qui est supportée de manière mobile par rapport à la cartouche d'agrafes chirurgicales de sorte que la surface de formation d'agrafe puisse être positionnée dans une relation de confrontation par rapport à la surface de plateau de cartouche d'agrafes (15311, 14911) ;
le dispositif d'agrafage étant conçu pour former la pluralité d'agrafes logées dans la cartouche d'agrafes (15300, 14900) contre la surface de formation d'agrafe de l'enclume (10060, 19200, 19600) en relation de confrontation par rapport à la surface de plateau d'agrafe (15311, 14911) de sorte que les agrafes soient formées avec des hauteurs formées différentes ;
**caractérisé par** un compensateur d'épaisseur de tissu (15120, 15220, 15320, 14920, 15020, 19010, 19030, 19610) positionné entre la surface de formation d'agrafe de l'enclume et la surface de plateau de cartouche d'agrafes et comprenant une épaisseur non uniforme mesurée depuis la surface de formation d'agrafe de l'enclume jusqu'à la surface de plateau de cartouche d'agrafes.

2. Dispositif d'agrafage chirurgical selon la revendication 1, la pluralité d'agrafes chirurgicales comprenant une pluralité de premières agrafes chirurgicales (15130a, 19540) ayant une première hauteur non formée et une pluralité de deuxièmes agrafes chirurgicales (15130b, 19542) ayant une deuxième hauteur non formée qui est différente de la première hauteur non formée.

3. Dispositif d'agrafage chirurgical selon la revendication 2 comprenant en outre une pluralité de troisièmes agrafes chirurgicales (15130c, 19544) ayant une troisième hauteur non formée qui diffère des première et deuxième hauteurs non formées.

4. Dispositif d'agrafage chirurgical selon la revendication 3, la cartouche d'agrafes chirurgicales comprenant en outre une fente allongée (19550) s'étendant à travers une partie de celle-ci pour recevoir un élément de coupe de tissu et les premières agrafes chirurgicales (19540) étant agencées dans des premières lignes d'agrafes chirurgicales situées sur chaque côté latéral de la fente et les deuxièmes agrafes chirurgicales (19542) étant agencées dans des deuxièmes lignes d'agrafes chirurgicales situées sur chaque côté latéral de la fente et chaque troisième agrafe chirurgicale (19544) étant agencée dans des troisièmes lignes d'agrafes chirurgicales situées sur chaque côté latéral de la fente.

5. Dispositif d'agrafage chirurgical selon l'une quelconque des revendications précédentes, la surface de plateau (14911) de la cartouche d'agrafes chirurgicales étant non plane et le compensateur d'épaisseur de tissu (14920) comprenant une partie de corps comprenant un profil de surface qui correspond sensiblement à la surface non plane de la surface de plateau.

6. Dispositif d'agrafage chirurgical selon la revendication 5, la partie de corps étant fabriquée à partir d'un matériau en mousse biocompatible.

7. Dispositif d'agrafage chirurgical selon la revendication 5 ou la revendication 6 comprenant en outre un film biocompatible perméable à l'air (19020) fixé à la partie de corps.

8. Dispositif d'agrafage chirurgical selon l'une quelconque des revendications précédentes, la surface de formation d'agrafe de l'enclume étant non plane et le compensateur d'épaisseur de tissu (19610) comprenant une partie de corps comprenant un profil de surface qui correspond sensiblement à la surface de formation d'agrafe non plane.
